# EUROPEAN PATENT SPECIFICATION

(11) **EP 1 930 723 B1**
(45) Date of publication and mention of the grant of the patent: **25.01.2017**
(21) Application number: 07022940.6
(22) Date of filing: 27.11.2007
(51) Int. Cl.: G01N 15/14, G01N 15/00, G01N 33/50

(54) **Method for measuring biological sample and measuring apparatus therefor**
Verfahren zur Messung von biologischen Proben und Messvorrichtung dafür
Procédé pour la mesure d'un échantillon biologique et appareil de mesure correspondant

(30) Priority: 27.11.2006 JP 2006318212
(43) Date of publication of application: 11.06.2008
(73) Proprietor: SYSMEX CORPORATION, Kobe-shi, Hyogo 651-0073 (JP)
(72) Inventor: Tsuji, Tomohiro, Kobe-shi Hyogo 651-0073 (JP); Yoshida, Ayumu, Kobe-shi Hyogo 651-0073 (JP); Oguni, Shinichiro, Kobe-shi Hyogo 651-0073 (JP)
(74) Representative: Hoffmann Eitle

(56) References cited:
- EP-A- 0 617 281
- EP-A- 0 867 720
- EP-A- 1 542 008
- EP-A- 1 813 942
- US-A- 5 958 776
- GREVE B ET AL: "High-grade loss of leukocytes and hematopoietic progenitor cells caused by erythrocyte-lysing procedures for flow cytometric analyses" JOURNAL OF HEMATOTHERAPY AND STEM CELL RESEARCH 200306 US, vol. 12, no. 3, June 2003 (2003-06), pages 321-330, XP009096886 ISSN: 1525-8165

## Description

### FIELD OF THE INVENTION

The present invention relates to a method for measuring a biological sample and in particular to a method for measuring a biological sample in which hematopoietic progenitor cells can be counted highly accurately by distinguishing them from other hemocytes.

### BACKGROUND

Measurement of hematopoietic progenitor cells in a biological sample is carried out in the clinical field. For example, hematopoietic progenitor cells in peripheral blood are measured in peripheral blood stem cell transplantation (referred to hereinafter as "PBSCT") known as therapy for leukemia and aplastic anemia. In PBSCT, a hematopoietic factor preparation (G-CSF) is administeredtoa healthy donor, whereby hematopoietic progenitor cells such as hematopoietic stem cells scarcely occurring in peripheral blood are allowed to appear in peripheral blood. After administration of G-CSF,hematopoieticprogenitor cells in peripheral blood are measured periodically, and when the number of hematopoietic progenitor cells appearing in peripheral blood reaches the maximum, the peripheral blood is collected by apheresis or the like. This peripheral blood thus collected is lyophilized and, when needed, is thawed and administered to a patient. Alternatively, autologous peripheral blood stem cell transplantation is carried out in which G-CSF is administered to a patient to allow hematopoietic progenitor cells to appear in peripheral blood, and the peripheral blood in which the hematopoietic progenitor cells have appeared is collected and administered to the patient himself.

In PBSCT, a sufficient amount of hematopoietic progenitor cells may not be obtained when collection of peripheral blood is too early or too late after administration of G-CSF.

Accordingly, techniques of accurately measuring hematopoietic progenitor cells are very important so as not to miss the timing of collection of peripheral blood. As a method for measuring hematopoietic progenitor cells, a technique disclosed in, for example, U.S. Patent No. 5830701 is known. According to U.S. Patent No. 5830701, a measurement sample of treated blood is first introduced into a flow cell of a flow cytometer, and its cells passing through the flow cell are irradiated with a light. Scattered light generated from the cells irradiated with the light is obtained, and on the basis of information on this scattered light, hematopoietic progenitor cells are identified.

EP1813942 discloses analyzing a blood sample by using a 2D scattergram based upon two axes of forward-scattered light intensity and fluorescence intensity to identify the total leucocyte region. Alternatively a 2D scattergram based upon 2 axes of side-scattered light intensity and fluorescence intensity is used. EP0867720 discloses counting hematopoietic progenitor cells in a sample by mixing the sample with a non-ionic water soluble surfactant, a solubilising agent, an amino acid and optionally a buffering agent. The authors propose to look at 2 items of cell information, such as e.g,. lateral scattered light and forward scattered light.

### SUMMARY

The present invention relates to a method for measuring a biological sample, comprising steps of:
preparing a measurement sample by damaging cell membranes of erythrocytes and mature leukocytes in a biological sample, substantially not damaging cell membranes of hematopoietic progenitor cells in the biological sample, shrinking the damaged erythrocytes, and staining nucleic acid in the damaged mature leukocytes with a fluorescent dye by mixing a surfactant for damaging cell membranes of erythrocytes and mature leukocytes, a solubilizing agent for shrinking the damaged erythrocytes, a fluorescent dye for staining a nucleic acid, and a biological sample for 3 seconds to 5 minutes, wherein the surfactant is a polyoxyethylene non-ionic surfactant represented by the formula: R¹-R²-(CH₂CH₂O)ₙ-H, wherein R¹ is a C₉ to C₂₅ alkyl, alkenyl or alkynyl group, R² is -O-, -COO- or 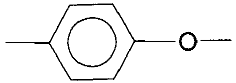 and n is an integer of 10 to 40;
irradiating cells in the measurement sample with light;
obtaining forward scattered light intensity, side scattered light intensity and fluorescence intensity from the irradiated cells;
specifying hematopoietic progenitor cells based on the obtained forward and side scattered light intensities and the obtained fluorescence intensity, comprising a first preparation of a first two-dimensional scattergram and a second preparation of a second two-dimensional scattergram; and
counting the specified hematopoietic progenitor cells;
characterized in that:
the first two dimensional scattergram has the forward and side scattered light intensities on the two axes and displays hematopoietic progenitor cells and platelet aggregate discriminated by the forward and side scattered light intensities; and
the second two dimensional scattergram has the forward or side scattered light intensity and the fluorescent intensity on the two axes and displays hematopoietic progenitor cells, granulocytes, lymphocytes, and monocytes discriminated by the forward or side scattered light intensity and the fluorescence intensity.

### BRIEF DESCRIPTION OF THE DRAWINGS

Fig. 1 is an illustration of a scattergram showing a first cell group.
Fig. 2 is an illustration of a scattergram showing a second cell group.
Fig. 3 is an example of a scattergram showing cells contained in a first cell group and also contained in a second cell group.
Fig. 4 is an illustration of a scattergram showing a third cell group.
Fig. 5 is an illustration of a scattergram showing a fourth cell group.
Fig. 6 is a view showing one example of the outward appearance of a measuring apparatus in this embodiment.
Fig. 7 is a view showing one example of a flow cytometer installed in a measuring apparatus in this embodiment.
Fig. 8 is a block diagram showing one example of the constitution of a control unit of a measuring apparatus in this embodiment.
Fig. 9 is a flowchart showing one example of processing in a control unit.
Fig. 10 is a flowchart showing another example of processing in a control unit.
Fig. 11 is a flowchart showing another example of processing in a control unit.
Fig. 12 is a flowchart showing another example of processing in a control unit.
Fig. 13 is a flowchart showing another example of processing in a control unit.
Fig. 14 is a scattergram A.
Fig. 15 is a scattergram B.
Fig. 16 is a graph showing the correlation between Example 1 and Comparative Example 1.
Fig. 17 is a scattergram C.
Fig. 18 is a scattergram D.
Fig. 19 is a scattergram E.
Fig. 20 is a scattergram F.
Fig. 21 is a graph showing the correlation between Example 2 and Comparative Example 2.
Fig. 22 is a graph showing the results of Example 3 and Comparative Example 3 where samples collected from subject A were used and measured.
Fig. 23 is a graph showing the results of Example 3 and Comparative Example 3 where samples collected from subject B were used and measured.

### DETAILED DESCRIPTION OF THE PREFERRED EMBODIMENTS

The preferred embodiments of the present invention are described hereinafter with reference to the drawings.

As used herein, the term "hematopoietic progenitor cell" (also referred to hereinafter as "HPC") refers generically to cells before blast cells in the differentiation stage in the hemocyte differentiation process of differentiating pluripotent stem cells into blood cells of each line. Specifically, the hematopoietic progenitor cells include pluripotent stem cells, lymphoid stem cells, myeloid stem cells, erythroid colony forming cells, megakaryocytic burst colony forming cells, neutrophil/macrophage colony forming cells, eosinophils colony forming cells, B-cell precursors, T-cell precursors or the like.

As used herein, the term "mature leukocytes" refers to mature lymphocytes, monocytes, granulocytes, neutrophils, eosinophils and basophils.

As used herein, "platelet aggregates" refer to particles each having 2 or more platelets aggregated therein.

As used herein, "damaging cell membranes" refers to formation of pores in cell membranes through which specific substances can pass.

As used herein, a "biological sample" refers mainly to peripheral blood, and preferable examples further include leukocyte component-containing biological samples such as bone marrow aspirate, a blood component-containing sample recovered by apheresis or the like, abdominal fluid and a urine sample.

Hematopoietic progenitor cells contained in a biological sample can be accurately classified and counted according to the present invention.

The method for measuring a biological sample according to the present invention comprises a step of treating a biological sample by damaging cell membranes of erythrocytes and mature leukocytes contained in the sample, shrinking hemocytes whose cell membranes have been damaged, and staining them with a fluorescent dye capable of staining a nucleic acid; a step of obtaining first scattered light information, second scattered light information based on a scattered light different in angle from the first scattered light, and fluorescence information, generated by irradiating the treated sample with a light; a step of identifying a cell group containing hematopoietic progenitor cells, discriminatively from other hemocytes, by using the obtained fluorescence information and two kinds of scattered light information; and a step of counting the identified cell group as hematopoietic progenitor cells.

Hereinafter, the present invention is described in more detail.

### Treatment of Biological Sample

The sample treatment step in the measurement method of the present invention is conducted preferably by mixing a biological sample with a treating reagent described later.

The treating reagent is a reagent capable of damaging cell membranes of erythrocytes and mature leukocytes contained in a biological sample, shrinking hemocytes whose cell membranes have been damaged, and staining at least a nucleic acid. Specifically, the treating reagent includes a surfactant for damaging cell membranes of erythrocytes and mature leukocytes contained in a sample, and a fluorescent dye capable of fluorescently staining at least a nucleic acid.

The surfactant used in the present invention damages cell membranes of erythrocytes and mature leukocytes. Although the mechanism of action is not evident, it is estimated that a part of specific cell membrane lipid components are extracted (drawn out) thereby forming pores (this is called damage) in cell membranes to such an extent that specific substances can pass therethrough. The fluorescent dye can penetrate into a cell damaged by the surfactant, thereby staining at least a nucleic acid therein.

The surfactant has an action of damaging young leukocytes as well, but requires a longer time in damaging young leukocytes than in damaging erythrocytes and mature leukocytes. Accordingly, within a predetermined time after mixing a biological sample with the treating reagent, damaged mature leukocytes are in a more easily stainable state than undamaged hematopoietic progenitor cells or young granulocytes. As a result, the hematopoietic progenitor cells or young granulocytes, as compared with the mature leukocytes damaged to stain their nuclei, are hardly stainable with the nucleus-staining fluorescent dye, thus exhibiting fluorescence intensity lower than that of the mature leukocytes.

The surfactant used in the present invention includes a polyoxyethylene nonionic surfactant represented by the formula: R1-R2-(CH₂CH₂O)ₙ-H wherein R1 is a C9 to C25 alkyl, alkenyl or alkynyl group, R2 is -O-, -COO- or 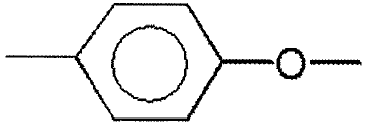 and
n is an integer of 10 to 40.

The C9 to C25 alkyl group includes nonyl, decyl, undecyl, dodecyl, tridecyltetradecyl and the like. The C9 to C25 alkenyl group includes dodecenyl, tetradecenyl and the like. The C9 to C25 alkynyl group includes dodecynyl, undecynyl, dodecynyl and the like.

Specific examples include polyoxyethylene (20) lauryl ether, polyoxyethylene (15) oleyl ether, polyoxyethylene (16) oleyl ether, polyoxyethylene (20) oleyl ether, and the like.

The surfactant can be used in the form of an aqueous solution. Although the concentration of a polyoxyethylene-based nonionic surfactant, for example, in water varies depending on the type of the surfactant used, theabove-mentionedpolyoxyethylene (16) oleyl ether can be used in the range of 5 to 50 g/l, preferably 15 to 35 g/l. The cell-damaging properties of the polyoxyethylene-based nonionic surfactant is increased with a lower value of n, and is decreased with a higher value of n, when the number of carbon atoms in its hydrophobic group is the same. When the value of n is the same, the cell-damaging properties is increased as the number of carbon atoms in the hydrophobic group is decreased. With this feature in view, the necessary concentration of the surfactant may be experimentally suitably determined using the above-mentioned values as a guide.

The fluorescent dye used in the present invention is a dye for fluorescently staining at least a nucleic acid. Mature leukocytes with damaged cell membranes, the fluorescent dye can pass through the cell membranes and bind to a nucleic acid, for example in the cells, thereby particularly staining their nuclei. A difference in fluorescence intensity between hematopoietic progenitor cells and other leukocytes is generated preferably by staining with this fluorescent dye.

Examples of the fluorescent dye include ethidium bromide, propidium iodide and products commercially available from Molecular Probe Ltd. , such as an ethidium-acridine heterodimer, ethidium azide, ethidium homodimer-1, ethidium homodimer-2, ethidium monoazide, TOTO-1, TO-PRO-1, TOTO-3, TO-PRO-3 or the like. When He-Ne, a red semiconductor laser or the like is used as a light source, it is possible to preferably use a dye represented by the following formula (1): 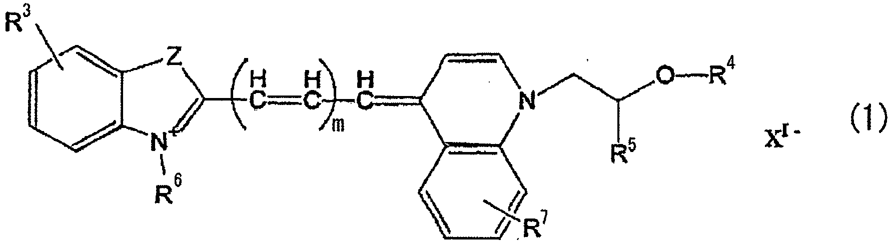 wherein R3 represents a hydrogen atom, a lower alkyl group or a lower alkoxy group, R4 represents a hydrogen atom, an acyl group or a lower alkyl group, R5 represents a hydrogen atom or an optionally substituted lower alkyl group, R6 represents a hydrogen atom or a lower alkyl group, R7 represents a hydrogen atom, a lower alkyl group or a lower alkoxy group, Z represents a sulfur atom, an oxygen atom or a lower alkyl group-substituted carbon atom, m is 1 or 2, and X is an anion.

The lower alkyl group represented by R3, R4, R5, R6, R7 or Z refers to a C1 to C6 linear or branched alkyl group including, for example, a methyl group, ethyl group, propyl group, butyl group, isobutyl group, sec-butyl group, tert-butyl group, pentyl group and hexyl group, among which a methyl group and an ethyl group are preferable.

The lower alkoxy group represented by R3 or R7 refers to a C1 to C6 alkoxy group including, for example, a methoxy group, ethoxy group and propoxy group, among which a methoxy group and an ethoxy group are preferable. R3 and R7 are preferably hydrogen atoms.

The acyl group represented by R4 is preferably an acyl group derived from an aliphatic carboxylic acid and includes, for example, acetyl, propionyl and the like, among which an acetyl group is preferable.

The optionally substituted lower alkyl group represented by R5 refers to a lower alkyl group which may be substituted with 1 to 3 hydroxyl groups, halogen atoms (fluorine, chlorine, bromine or iodine)or the like, among which a methyl or ethyl group substituted with 1 hydroxyl group is preferable.

The group represented by Z is preferably a sulfur atom.

Examples of the anion X⁻ include halogen ions (fluorine, chlorine, bromine and iodine ions), borohalide ions (forexample, BF₄⁻, BCl₄⁻, BBr₄⁻), phosphorous compound ions, halooxy-acid ions, fluorosulfate ions, methyl sulfate ions, and tetraphenylboron compound ions whose aromatic ring is substituted with a halogen or a halogen-containing alkyl group. In particular, a bromine ion or BF₄⁻ is preferable.

Preferable examples of the dye represented by the formula (I) above are the following dyes A, B and C:

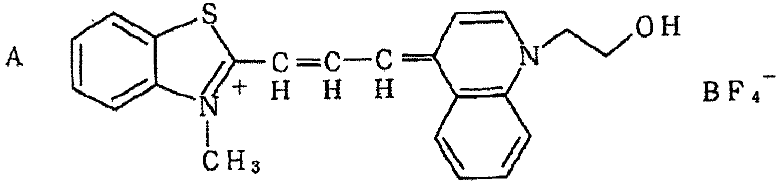

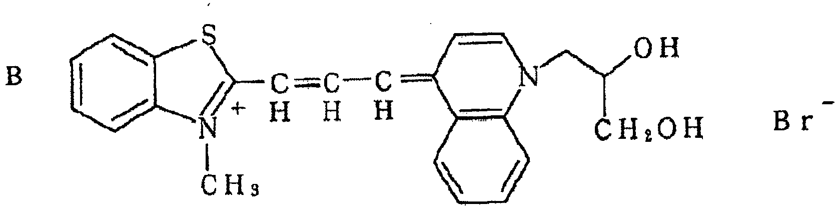

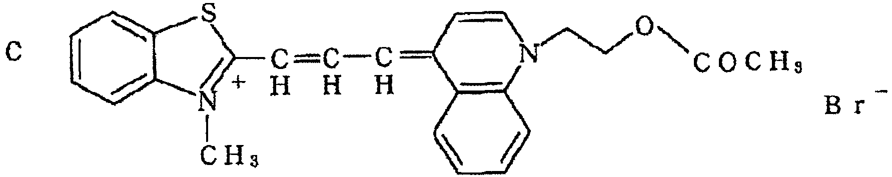

The concentration of a dye can be suitably determined depending on the dye used. The concentration of the dye in the reagent is preferably 0.01 to 500 ppm, more preferably 0.1 to 200 ppm.

The cell membrane is damaged by the above surfactant, thereby discharging a part of intracellular fluid out of the cell to cause cell shrinkage or changing the surface state of the cell membrane to cause cell shrinkage. The treating reagent preferably contains a solubilizing agent for sufficiently shrinking damaged cells, thus allowing hemocytes (particularly erythrocytes) other than leukocytes to appear as a ghost group not providing useful scattered light information and fluorescence information.

As the solubilizing agent, it is possible to use one or more members selected from the group consisting of:
sarcosine derivatives represented by the following formula (2) or salts thereof: 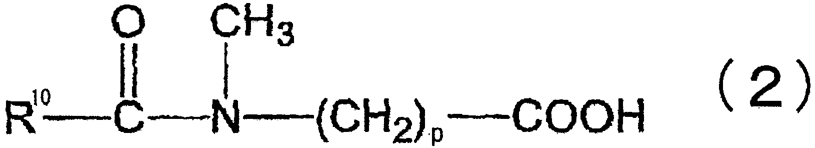 wherein R¹⁰ represents a C10 to C22 alkyl group, and p is an integer of 1 to 5;
cholic acid derivatives represented by the following formula (3): 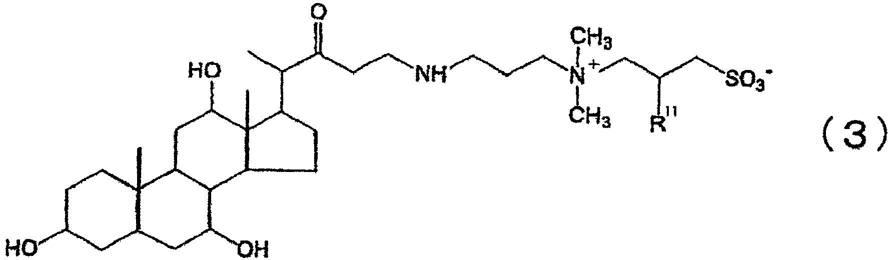 wherein R¹¹ represents a hydrogen atom or a hydroxyl group; and
methyl glucan amides represented by the following formula (4) : 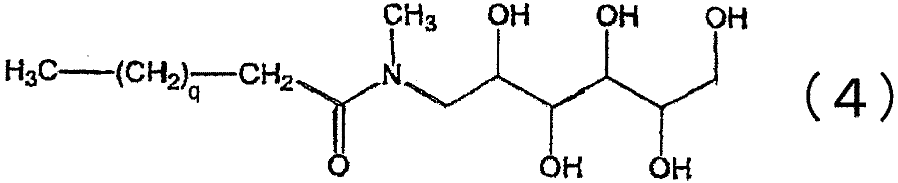 wherein q is 5 to 7.

The C10 to C22 alkyl group includes decyl, dodecyl, tetradecyl, oleyl and the like.

Specific examples of the solubilizing agent include N-lauroyl sarcosine sodium, lauroyl methyl β-alanine sodium, lauroyl sarcosine, CHAPS (3-[3-choleamidopropyl) dimethylammonio]-1-propansulfonate), CHAPSO (3-[3-choleamidopropyl) dimethylammonio]-2-hydroxy-1-propansulfonate), MEGA8 (octanoyl-N-methylglucamide), MEGA9 (nonanoyl-N-methylglucamide), MEGA10 (decanoyl-N-methylglucamide) and the like.

The concentration of the sarcosine acid derivative or salts thereof as the solubilizing agent is preferably 0.2 to 2.0 g/l, the concentration of the cholic acid derivative is preferably 0.1 to 0.5 g/l, and the concentration of the methyl glucan amide is preferably 1.0 to 8.0 g/l.

Besides those described above, n-octyl β-glucoside, sucrose mono capcaprate, N-formyl methyl leucyl alanine and/or the like may be used as the solubilizing agent and used preferably at a concentration of 0.01 to 50.0 g/l.

A preferable pH of the reagent is 5.0 to 9.0. For regulating the pH in this range, a buffer agent is preferably contained in the reagent. As the buffer agent, Good buffers such as HEPES and the like and phosphate buffers are used, and sodium hydroxide, for example, are used for regulating the pH.

The osmotic pressure of the reagent is regulatedpreferably at 50 to 600 mOsm/kg, more preferably 50 to 300 mOsm/kg. For regulating the osmotic pressure of the reagent in the desired range, an osmotic pressure-regulating agent is preferably contained in the reagent. A sugar, an amino acid, an organic solvent, sodium chloride and the like are used as the osmotic regulating agent. As the sugar, glucose, xylitol, mannitol, arabinose, ribitol or the like can be used. As the amino acid, alanine, proline, glycine, valine and the like can be used. As the organic solvent, ethylene glycol, glycerin and the like can be used. The concentration of the osmotic pressure-regulating agent in the reagent is regulated suitably depending on its molecular weight; for example, when xylitol is used, its concentration is preferably 10 to 75 g/l; when glycerin is used, its concentration is preferably 5 to 45 g/l; and when glycine is used, its concentration is preferably 5 to 45 g/l.

The treating reagent may be a reagent of one-pack type containing a surfactant, a dye and other components, or may be formed into a reagent kit provided with the fluorescent dye-containing dyeing solution and the surfactant-containing solution (hemolytic agent) which are stored in separate containers. In this case, a water-soluble organic solvent such as ethylene glycol is preferably used as a solvent for the fluorescent dye in order to increase the storage stability of the dye.

### Preparation of Measurement Sample

By mixing a solution containing the treating reagent with a biological sample, a measurement sample can be prepared. When the reagent kit described above is used, the surfactant-containing solution (hemolytic agent) may be first mixed with a biological sample and then mixed with the dyeing solution to prepare a measurement sample.

The biological sample and the treating reagent are mixed preferably at a sample : treating reagent ratio of from 1 : 10 to 1 : 1000. The temperature at which the sample is reacted with the treating reagent is preferably 20 to 40°C. The time for which the sample is reacted with the treating reagent is 3 seconds to 5 minutes, more preferably 4 seconds to 1 minute.

In the measurement sample thus prepared, the sizes of erythrocytes and mature leukocytes are reduced due to shrinkage of the cells. Particularly, erythrocytes lack nuclei and thus have undergone the progress of hemolysis and cell shrinkage due to damage to cell membranes, and are hardly stained with the fluorescent dye, thus reducing their forward scattered light, side scattered light and fluorescence thereby allowing the erythrocytes to be handled as a ghost group.

Mature leukocytes, on the other hand, are shrunk upon damage to their cell membranes by reaction of the biological sample with the reagent for a predetermined time, but because their nuclei are substantially not shrunk, the mature leukocytes are not so shrunk as erythrocytes. Further, the fluorescent dye can pass through the damaged membranes so that at least nuclei of the mature leukocytes are stained.

Hematopoietic progenitor cells are more resistant to the hemolytic agent than mature leukocytes and erythrocytes, thus requiring a longer time for damage to their cell membranes. Accordingly, hematopoietic progenitor cells are substantially free from cell shrinkage within a predetermined reaction time, and further the fluorescent dye cannot pass therethrough, so their nuclei are hardly stained.

Similar to the hematopoietic progenitor cells, platelets contained in the sample require a longer time in damaging their membranes by the treating reagent. Although the dye does not penetrate into platelets, the platelets are stained by physical adsorption of the dye around their aggregates.

### Acquisition of Scattered Light Information and Fluorescence Information

The prepared measurement sample was introduced into a flow cytometer where scattered light information and fluorescence information on cells, platelets etc. contained in the sample can be obtained.

The information is obtained preferably by introducing the measurement sample into a flow cell of a flow cytometer and irradiating the sample flowing in the flow cell with an exciting light capable of exciting the fluorescent dye. The scattered light information and fluorescence information emitted from the cells passing through the flow cell can thereby be obtained.

The irradiating light contains a light of wavelength capable of exciting the fluorescent dye used. Depending on the wavelength of a light from a light source installed in the flow cytometer used, a fluorescent dye which can be excited with the light may be selected.

As the scattered light information, two kinds of scattered light information different in angle are preferably obtained. Specifically, a combination of forward scattered light and side scattered light is preferably used.

The forward scattered light information is considered as information reflective of cell size. That is, when a cell is large, its forward scattered light is high. The side scattered light is a scattered light different in angle by 80 to 100°, preferably by 85 to 95°, from the forward scattered light. From the side scattered light, information on the inside of the cell, particularly on its nucleus and granules, can be obtained. When granules are contained or the shape of nucleus is distorted, the side scattered light tends to increase.

Within a predetermined reaction time, the fluorescent dye hardly penetrates into platelets, and thus the platelets themselves are hardly stained. However, the fluorescent dye binds to surrounding areas of platelet aggregates and to regions where platelets adhere tightly to one another, so that in the measurement sample, the fluorescence intensity is increased as the aggregates are enlarged. On the other hand, when platelet aggregates are few and the aggregates are small, the side scattered light intensity is decreased, and the fluorescent dye adhering thereto is reduced, resulting in lower fluorescence intensity.

### Identification of Cell Group and Counting of Hematopoietic Progenitor Cells

Using the obtained scattered light information and fluorescence information, the objective cell group is identified and hematopoietic progenitor cells are counted. The measurement method of the present invention can be classified into the following first to fifth measurement methods. Hereinafter, the first to fifthmeasurementmethods are described in this order.

### (1) First Measurement Method

In the first measurement method, a first cell group containing hematopoietic progenitor cells is identified on the basis of the obtained first scattered light information and second scattered light information (first identification step), a second cell group containing hematopoietic progenitor cells is identified on the basis of the obtained first scattered light information and fluorescence information (second identification step), and cells contained in the first cell group and also contained in the second cell group are counted as hematopoietic progenitor cells. It does not matter whichever of the first and second identification steps is first conducted.

### (1-1) First Cell Group

The first cell group is identified on the basis of 2 kinds of obtained scattered light information, preferably on the basis of forward scattered light intensity and side scattered light intensity. Preferably, the first cell group is identified by establishing an area where hematopoietic progenitor cells are estimated to appear in a scattergram (first scattergram) with the 2 kinds of scattered light information on the two axes. Preferably, the establishment of the area where the first cell group appears is conducted for each sample because the staining and size of the hematopoietic progenitor cell vary from sample to sample.

By way of example, a specific method for establishing the area where the first cell group appears is illustrated. When a cell group is recognized in a position where hematopoietic progenitor cells are estimated to appear in a scattergram with the 2 kinds of scattered light information on the two axes, the center of this group is first specified. A section ranging from the center of the cell group of hematopoietic progenitor cells to an area where hematopoietic progenitor cells appear without another cell group appearing therein can be determined as the area where the first cell group appears. Similarly, when cell groups are recognized in positions where other cell groups (young granulocytes, granulocytes, lymphocytes, monocytes) are estimated to appear, the centers of the respective groups are specified and simultaneously the areas where the respective groups appear are specified.

These areas have previously been established in a scattergram, from accumulated data on hematopoietic progenitor cells of various sizes with various dyes and concentrations used.

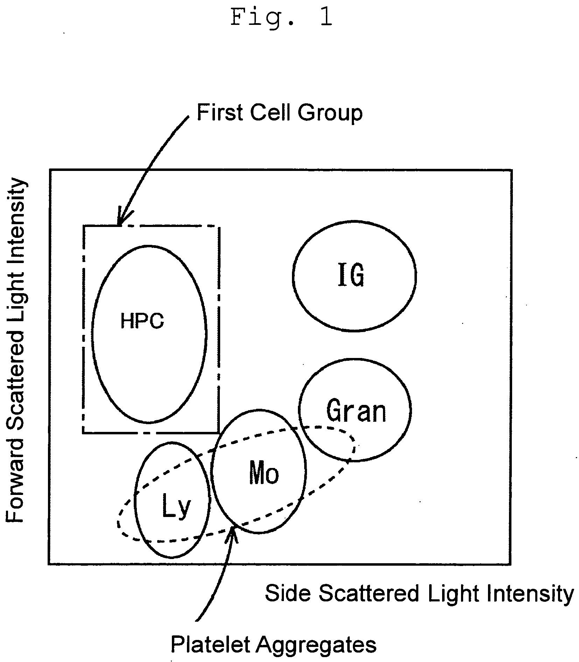

For example, when the areas where the respective cells are estimated to appear are established in a scattergram with two axes indicating forward scattered light intensity (first scattered light information) on the ordinate and side scattered light intensity (second scattered light information) on the abscissa, Fig. 1 is typically given. In Fig. 1, "HPC" is a region where hematopoietic progenitor cells appear, "IG" is a region where young granulocytes appear, "Gran" is a region where granulocytes appear, "Ly" is a region where lymphocytes appear, and "Mo" is a region where monocytes appear.

Because of lower side scattered light intensity, hematopoietic progenitor cells will appear in a relatively left-side side region in the scattergram. On the other hand, granulocytes and young granulocytes will, because of distorted shape of their nuclei, appear in right-side regions where side scattered light intensity is relatively high, and thus these groups will appear in regions distant from the region of hematopoietic progenitor cells. Accordingly, the region indicated by an alternate long and short dash line enclosing the HPC region may be established as the region where the first cell group appears.

Platelet aggregates show side scattered light intensity varying depending on a manner in which the aggregates are formed. The size of an aggregate varies widely from a few of platelets to few dozens of platelets, so the platelet aggregates also show varying forward scattered light intensity. However, the size of an aggregate is correlated with the distortion of the aggregate so that in the scattergram shown in Fig. 1, platelet aggregates appear in a lower left to upper right region (region enclosed with a dotted line), and this region will not overlap with the HPC region (region where the first cell group appears).

According to Fig. 1, the first cell group is distinguished from platelet aggregates and other hemocyte components, and thus it is anticipated that the first cell group can be counted as hematopoietic progenitor cells. However, lymphocytes of large size may, depending on the sample, be contained in that region, and thus a part of the region where lymphocytes appear can, in rare cases, overlap with the region where the first cell group appears.

### (1-2) Second Cell Group

The second cell group is identified on the basis of the obtained first scattered light information and fluorescence information, preferably on the basis of forward scattered light information and fluorescence information. The second cell group is a cell group contained in the region where hematopoietic progenitor cells are estimated to appear in a scattergram (second scattergram) with forward scattered light intensity and fluorescence intensity on the two axes.

Within a predetermined reaction time, hematopoietic progenitor cells and young granulocytes are substantially free from the damage to cell membranes by the treating reagent, and are thus not stained and will appear in a left-side region where fluorescence intensity is low. Accordingly, in the region where hematopoietic progenitor cells are estimated to appear, young leukocytes may appear in some cases. On the other hand, granulocytes, lymphocytes and monocytes undergo membrane damage by the treating reagent in a predetermined reaction time thereby allowing their nuclei to be stained, and will thus appear in a right-side region where fluorescence intensity is high, and therefore, these cells can be clearly distinguished from young leukocytes whose nuclei are not substantially stained in a predetermined reaction time. That is, mature leukocytes do substantially not appear in the region where young leukocytes containing hematopoietic progenitor cells appear.

By way of example, a specific method for specifying the area where the second cell group appears is illustrated. When a cell group is recognized in a position where hematopoietic progenitor cells are estimated to appear in a scattergram, the center of this group is specified. A section ranging from the center of the cell group of hematopoietic progenitor cells to an area where hematopoietic progenitor cells appear without a cell group of mature leukocytes appearing therein can be determined as the area where the second cell group appears. Similarly, when cell groups are recognized in positions where other cell groups (young granulocytes, granulocytes, lymphocytes, monocytes) are estimated to appear, the centers of the respective groups are specified and simultaneously the areas where the respective groups appear are specified. These areas have previously been established from accumulated data on hematopoietic progenitor cells of various sizes with various dyes and concentrations used.

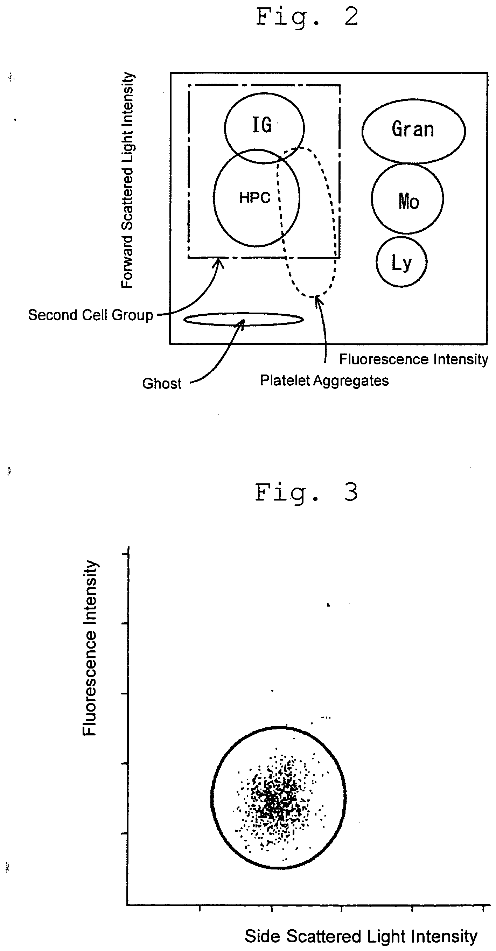

Specifically in a scattergram with fluorescence intensity on the abscissa and forward scattered light intensity on the ordinate as shown in Fig. 2, the second cell group is a group contained in a left-side region of low fluorescence intensity as enclosed with an alternate long and short dash line. In Fig. 2, "HPC" is a region where hematopoietic progenitor cells appear, "IG" is a region where young granulocytes appear, "Gran" is a region where granulocytes appear, "Ly" is a region where lymphocytes appear, and "Mo" is a region where monocytes appear.

Staining of platelet aggregates is caused by mere physical adsorption and thus the aggregates appear in a left-side region. Platelet aggregates larger or smaller than hematopoietic progenitor cells can exist depending on the degree of aggregation and thus appear in a vertically long region of low to high forward scattered light intensity.

### (1-3) Counting of Hematopoietic Progenitor Cells

The cells contained in the first cell group and also contained in the second cell group as identified as described above are counted as hematopoietic progenitor cells.

Lymphocytes which may be contained in the first cell group are not contained in the second cell group. On the other hand, platelet aggregates and young leukocytes which may be contained in the second cell group are not contained in the first cell group. Accordingly, it can be said that the cells contained in the first cell group and also contained in the second cell group are free of lymphocytes, platelet aggregates and young leukocytes and are substantially hematopoietic progenitor cells. Hence, the cells contained in the first cell group and also contained in the second cell group can be counted as hematopoietic progenitor cells, thereby accurately counting hematopoietic progenitor cells.

The cells contained in the first cell group and also contained in the second cell group may be developed in a scattergram (third scattergram) with fluorescence information and second scattered light information on the two axes. Cells appearing on the third scattergram consist substantially of hematopoietic progenitor cells.

The first scattered light information is preferably forward scattered light intensity, and the second scattered light information is preferably side scattered light intensity. When the cells contained in the first cell group and also contained in the second cell group are developed in a scattergram (third scattergram) with fluorescence intensity and side scattered light intensity on the two axes, a scattergram shown in, for example, Fig. 3 is given. In this scattergram, the cells contained in the first cell group and also contained in the second cell group, that is, a cell group consisting essentially of hematopoietic progenitor cells appears, and thus the cells appearing therein can be counted as hematopoietic progenitor cells.

### (2) Second Measurement Method

In the first measurement method, the first identification step and the second identification step are separately conducted whereby the first cell group and the second cell group are identified respectively. Alternatively, the first (or second) cell group may be first identified followed by identifying the second (or first) cell group from among the previously identified first (or second) cell group.

That is, in the second measurement method, the second cell group containing hematopoietic progenitor cells is identified on the basis of the obtained first scattered light information and fluorescence information (second identification step), and then the first cell group is identified from among the second cell group, on the basis of the first scattered light information and second scattered light information of the identified second cell group (this identification step when referred to specifically in the second measurement method is referred to as "first' identification step", and the first cell group identified in the first' identification step is referred to as "first' cell group"), and the first' cell group is counted as hematopoietic progenitor cells. Alternatively, the first cell group containing hematopoietic progenitor cells is identified on the basis of the obtained first scattered light information and second scattered light information (first identification step), and then the second cell group is identified from among the first cell group, on the basis of the first scattered light information and fluorescence information of the identified first cell group (this identification step when referred to specifically in the second measurement method is referred to as "second' identification step", and the second cell group identified in the second' identification step is referred to as "second' cell group"), and the second' cell group is counted as hematopoietic progenitor cells.

The first identification step and the first cell group, and the second identification step and the second cell group, are identified respectively on the basis of the scattered light information and fluorescence information obtained in the information acquisition step, and the specific method therefor is the same as described above in the first measurement method, so its description is omitted.

### (2-1) Identification of the Second' Cell Group and Counting of Hematopoietic Progenitor Cells

In the second' identification step, a group containing hematopoietic progenitor cells is identified (identification of the second' cell group) from among the first cell group, on the basis of the first scattered light information (preferably forward scattered light intensity) and fluorescence information (preferably fluorescence intensity) of the previously identified first cell group. The second' cell group thus identified corresponds to the cells which in the first measurement method, are contained in the first cell group and also contained in the second cell group.

Identification of the second' cell group is conducted specifically in the following manner.

First, a scattergram (first scattergram) with the first scattered light information and second scattered light information on the axes is prepared, and on this scattergram, all concrete components (cells, platelets, for example) are developed in this scattergram. A candidate region where hematopoieticprogenitor cells appear is established in the first scattergram, and the first cell group appearing in this region is identified. Then, a scattergram (second' scattergram) with the first scattered light information and fluorescence information on the two axes is prepared, and only the first cell group is developed in this scattergram. The cell group contained in the region where hematopoietic progenitor cells are estimated to appear in the second' scattergram is the second' cancer group. In the second' scattergram with the first scattered light information and fluorescence information on the two axes, mature leukocytes are clearly distinguished from young leukocytes so that even if lymphocytes are contained in the first cell group, lymphocytes are not contained in the second' cell group developed in the second' scattergram. That is, the second' cell group is a group of hematopoietic progenitor cells substantially free of other components such as platelet aggregates and lymphocytes. Accordingly, the cells contained in the second' cell group may be counted as hematopoietic progenitor cells.

### (2-2) Identification of First' Cell Group and Counting of Hematopoietic Progenitor Cells

In the first' identification step, a second cell group is previously identified in the second identification step, and a group containing hematopoietic progenitor cells is identified from among the second cell group (identification of the first' cell group), on the basis of the first scattered light information (preferably forward scattered light intensity) and second scattered light information (preferably side scattered light intensity) of the identified second cell group. The first' cell group thus identified corresponds to the cells which in the first and second measurement methods, are contained in the first cell group and also contained in the second cell group.

Identification of the first' cell group is conducted specifically in the following manner.

First, a scattergram (second scattergram) with the obtained first scattered light information (preferably forward scattered light intensity) and fluorescence information (preferably fluorescence intensity) on the two axes is prepared, and all concrete components (cells, platelets, for example) are developed in this scattergram. A candidate region where the second cell group containing hematopoietic progenitor cells appears is specified in the second scattergram. Then, a scattergram (first' scattergram) with the first scattered light information and second scattered light information on the two axes is prepared, and only the second cell group specified above is developed in this scattergram. The cell group contained in the region where hematopoietic progenitor cells are estimated to appear in the first' scattergram is the first' cancer group. In the first' scattergram with the first scattered light information and second scattered light information on the two axes, young leukocytes and platelet aggregates appear in areas which can be clearly distinguished from the area where hematopoietic progenitor cells appear, and thus the first' cell group is substantially free of other components such as platelet aggregates, lymphocytes and young leukocytes. Accordingly, the cells contained in the first' cell group may be counted as hematopoietic progenitor cells.

### (3) Third Measurement Method

In the first and second measurement methods, the first cell group (or the first' cell group) and the second cell group (or the second' cell group) are separately identified on the basis of 2 kinds of information in either identification step, and the cell group contained in the first cell group and also contained in the second cell group is identified and counted as hematopoietic progenitor cells. In the third measurement method, the obtained first scattered light information, second scattered light information and fluorescence information are used to identify the first and second cell groups in one step, and the cells contained in both the first and second cell groups may be determined and counted as hematopoietic progenitor cells.

That is, in the third measurement method, the first cell group containing hematopoietic progenitor cells is identified on the basis of the obtained first scattered light information and second scattered light information, and simultaneously the second cell group containing hematopoietic progenitor cells is identified on the basis of the obtained first scattered light information and fluorescence information, and the cells contained in the first cell group and also contained in the second cell group are counted as hematopoietic progenitor cells.

Specifically, a sample is developed in a three-dimensional scattergram with first scattered light information, second scattered light information and fluorescence information on the three axes, and the first cell group containing hematopoietic progenitor cells is identified on the basis of the first scattered light information (preferably forward scattered light intensity) and the second light scattered light information (preferably side scattered light intensity), and simultaneously the second cell group containing hematopoietic progenitor cells is identified on the basis of the first scattered light information (preferably forward scattered light intensity) and the fluorescence information (preferably fluorescence intensity). The method for identifying the first cell group and the method for identifying the second cell group can be carried out in the same manner as in the first measurement method. In short, identification of the first cell group and identification of the second cell group can be carried out simultaneously in the three-dimensional scattergram, and further the cells contained in the first cell group and also contained in the second cell group can be identified. As described above in the first measurement method, the cells contained in the first cell group and also contained in the second cell group are substantially free of other cell components such as platelet aggregates and lymphocytes. Accordingly, when the cells contained in the first cell group and also contained in the second cell group, in the 3-dimensional scattergram, are counted as hematopoietic progenitor cells, the hematopoietic progenitor cells can be accurately counted.

### (4) Fourth Measurement Method

The fourth measurement method is a method applied to the case where in place of the second group containing hematopoietic progenitor cells, a third cell group containing cells (which are substantially all leukocytes because erythrocytes have become a ghost group by cell shrinkage in the measurement sample used in the present invention) other than the ghost group is identified as the cell group identified on the basis of the obtained first scattered light information and fluorescence information.

That is, in the fourth measurement method, a first cell group containing hematopoietic progenitor cells is identified on the basis of the obtained first light scattered light information and second light scattered light information (first identification step), a third cell group containing all leukocytes is identified on the basis of the obtained first scattered light information and fluorescence information (third identification step), a fourth cell group substantially free of mature leukocytes is identified from among the third cell group, on the basis of the second scattered light information and fluorescence information of the third cell group (fourth identification step), and the cells contained in the first cell group and also contained in the fourth cell group is counted.

The first identification step and the first cell group are the same as in the first measurement method, and their description is omitted.

### (4-1) Third Identification Step and Third Cell Group

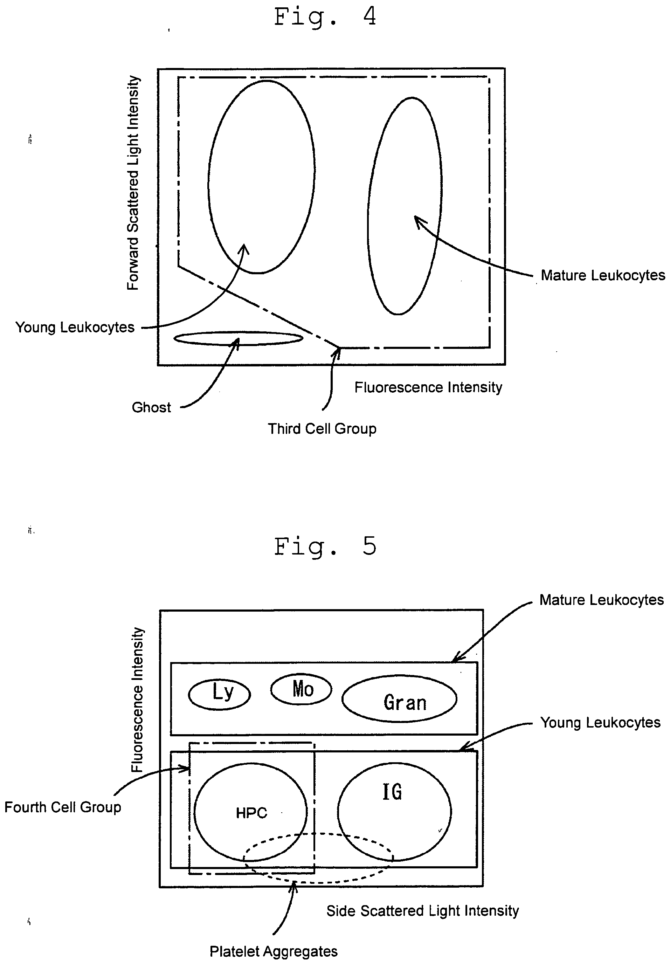

The third cell group is a group of substantially all leukocytes, that is, a cell group appearing in a region, excluding a region where an erythrocyte ghost group appears, in a scattergram with forward scattered light intensity and fluorescence intensity on the two axes. Specifically, when a scattergram with florescence intensity on the abscissa and forward scattered light intensity on the ordinate is drawn, the cell group appearing on the region enclosed with an alternate long and short dash line is a third cell group, as shown in Fig. 4. In the region where the third cell group appears, platelet aggregates are also contained as shown in Fig. 2.

### (4-2) Fourth Identification Step and Fourth Cell Group

A fourth cell group is identified on the basis of the second scattered light information and fluorescence information of the third cell group, preferably the side scattered light information and fluorescence information thereof. When the third cell group is developed in the scattergram with fluorescence intensity and side scattered light intensity on the two axes, the region where mature leukocytes appear can be clearly distinguished from the region where young leukocytes appear, and thus a cell group containing young leukocytes and a group containing hematopoietic progenitor cells can be identified by identifying the region considered to contain hematopoietic progenitor cells, in the scattergram with fluorescence intensity and side scattered light intensity on the two axes. Identification of a candidate region where hematopoietic progenitor cells appear is conducted preferably for each sample, similarly to identification of the first cell group or the second cell group. These regions may have previously been established from accumulated data on hematopoietic progenitor cells and mature leukocytes of various sizes with dyes and concentration used.

When the third cell group is developed in a scattergram with side scattered light intensity on the abscissa and fluorescence intensity on the ordinate, mature leukocytes (lymphocytes, monocytes and granulocytes) appear in upper regions because of staining of their nuclei as shown in Fig. 5, and can be distinguished clearly from young leukocytes with substantially unstained nuclei. Depending on the shape of nuclei, for example, the mature leukocytes appear as a lymphocyte group (Ly), a monocyte group (Mo) and a granulocyte group (Gran) from the left to right. Platelet aggregates appear in a lower region extending from the left to right. Accordingly, the cell group containing hematopoietic progenitor cells positioned in a region of relatively low to moderate fluorescence intensity in a left-side region of low side scattered light intensity as enclosed with an alternate long and short dash line may be identified as a fourth cell group. The hematopoietic progenitor cells and young granulocytes are different from each other in nuclear form and complexity and can thus be distinguished from each other by the degree of side scattered light intensity, but when gating for clearly distinguishing both of them from each other is difficult, the cell group contained in a young granulocyte-appearing region in which both hematopoietic progenitor cells and young granulocytes are contained may be a fourth cell group.

### (4-3) Identification and Counting of Hematopoietic Progenitor Cells

The cells contained in the first cell group and also contained in the fourth cell group as identified in the manner as described above are counted. The first cell group is free of platelet aggregates, while the fourth cell group is free of mature leukocytes, and thus the cells contained in the first cell group and also contained in the fourth cell group are a group of hematopoietic progenitor cells or young leukocytes which are substantially free of platelet aggregates and mature leukocytes. That is, even if lymphocytes are contained in the first cell group, the cells contained in the first cell group and also contained in the fourth cell group have been deprived of lymphocytes, so substantially hematopoietic progenitor cells or young leukocytes can be counted highly accurately.

### (5) Fifth Measurement Method

In the fifth measurement method, the third identification step in the fourth measurement method is carried out to identify a third cell group, and the fourth identification step is carried out to identify a fourth cell group from among the third cell group, and the fourth cell group is counted as hematopoietic progenitor cells. The third identification step and the third cell group are as described in (4-1) above, and the fourth identification step and the fourth cell group are as described in (4-2) above.

In the fifth measurement method, the fourth cell group is regarded as hematopoietic progenitor cells, so there is a possibility that the influence of platelet aggregates cannot be completely eliminated. However, when the amount of hematopoietic progenitor cells after administration of G-CSF is monitored, a large amount of hematopoietic progenitor cells is mobilized into peripheral blood, thus making it only necessary to know the rough number of hematopoietic progenitor cells and making the amount of platelet aggregates negligible. In such cases, therefore, the fifth measurement method not requiring such highly accurate measurement as in the first to fourth measurement methods can also be preferably used.

### Discrimination Method

In the present invention, the method for discriminating between hematopoietic progenitor cells and platelet aggregates contained in a biological sample is a method comprising steps of: treating a sample according to the measurement method of the present invention; obtaining forward scattered light information and side scattered light information generated by irradiating the treated sample with a light; and identifying a first cell group containing hematopoietic progenitor cells, on the basis of the forward scattered light information and the side scattered light information, thereby discriminating between the cell group containing hematopoietic progenitor cells and platelet aggregates.

As described above, when a region where hematopoietic progenitor cells are estimated to appear is identified in a scattergram with forward scattered light information and side scattered light information on the two axes, the region is distinguished clearly from a region where platelet aggregates are considered to appear, and thus hematopoietic progenitor cells and platelet aggregates contained in a biological sample can be discriminated from each other.

### Apparatus for Measuring Biological Sample

The apparatus usded for the present invention comprises a means for treating a biological sample by damaging cell membranes of erythrocytes and mature leukocytes contained in the sample, shrinking hemocytes whose cell membranes have been damaged, and staining them with a fluorescent dye capable of staining a nucleic acid; a means for obtaining first scattered light information, second scattered light information based on a scattered light different in angle from the first scattered light, and fluorescence information, generated by irradiating the treated sample with a light; a first identification means for identifying a first cell group containing hematopoietic progenitor cells, on the basis of the first scattered light information and the second scattered light information; a second identification means for identifying a second cell group containing hematopoietic progenitor cells, on the basis of the first scattered light information and the fluorescence information; and a means for counting, as hematopoietic progenitor cells, cells contained in the first cell group and also contained in the second cell group.

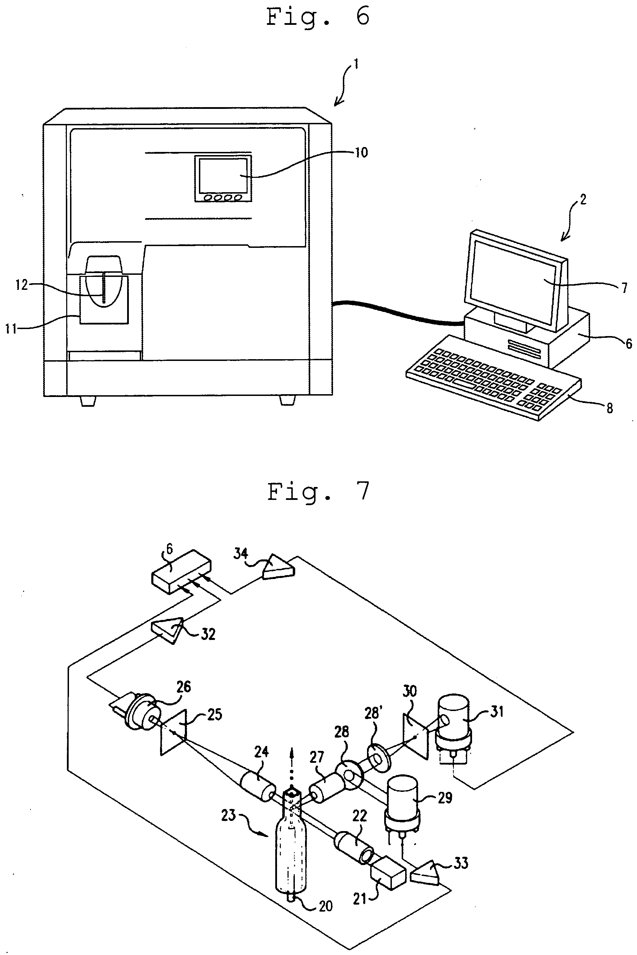

The measuring apparatus has an outward appearance shown in, for example, Fig. 6.

The apparatus shown in Fig. 6 has a measurement unit 1, an analysis unit 2, and a signal cable 3 connecting the analysis unit 2 to the measurement unit 1. The sample-treating means and information obtaining means in the apparatus are contained in the measurement unit 1, and the first identification means, the second identification means and the counting means are contained in the analysis unit 2.

The measurement unit 1 is provided with a liquid crystal touch panel 10 for input setting by the operator, a start switch 11 for initiating measurement operation, and a probe 12 for suctioning a sample. A flow cytometer as shown in Fig. 7 is installed in the measurement unit 1. The analysis unit 2 is provided with a control unit 6 for processing information sent from the measurement unit 1 and for controlling the operation of the measurement unit 1, an output unit 7 serving as a display for displaying various measurement results, and a keyboard 8 for inputting sample information and others by the operator.

Now, the flow cytometer shown in Fig. 7 is described.

A beam emitted from a light source 21 (for example, a red semiconductor laser: wavelength 633 nm) is used to irradiate an orifice of a sheath flow cell 23 via a collimating lens 22. Forward light scattered light emitted fromhemocytes which after discharge from a nozzle 20, has passed through the orifice, enters a forward scattered light detector (photodiode) 26 via a collecting lens 24 and a pinhole plate 25. On the other hand, side scattered light emitted from hemocytes passing through the orifice enters a side scattered light detector (photomultiplier tube) 29 via a collecting lens 27 and a dichroic mirror 28. Side fluorescence emitted from hemocytes passing through the orifice enters a side fluorescence detector (photomultiplier tube) 31 via the collecting lens 27, the dichroic mirror 28, a filter 28' and a pinhole plate 30. A forward scattered light signal outputted from the forward scattered light detector 26, a side scattered light signal outputted from the side scattered light detector 29, and a side fluorescence signal outputted from the side fluorescence detector 31 are amplified with amplifiers 32, 33 and 34 respectively and inputted into the control unit 6.

The light source 21 is a device which irradiates an orifice of a flow cell into which a prepared measurement sample was introduced, with a light capable of exciting a dye used in treatment of the sample and which is selected so as to meet the fluorescent dye staining hemocytes in the sample. Accordingly, a red semiconductor laser or others including, for example, an argon laser, a He-Ne laser, a bluer semiconductor laser and the like may also be used depending on the type of the fluorescent dye used.

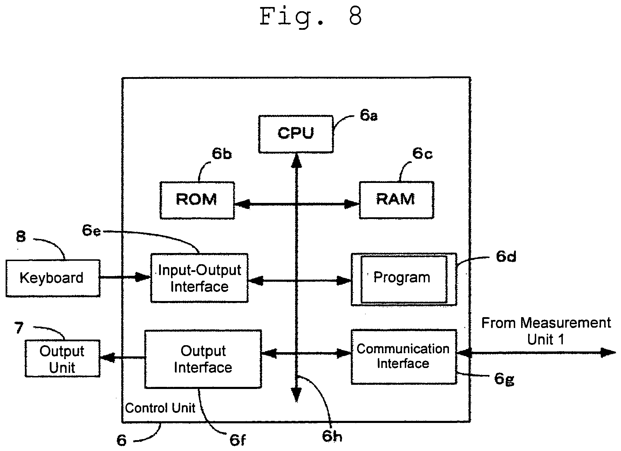

Fig. 8 is a block diagram showing the constitution of the control unit 6. The control unit 6 is composed mainly of CPU 6a, ROM 6b, RAM 6c, hard disk 6d, input-output interface 6e, image output interface 6f and communication interface 6g, and the CPU 6a, ROM 6b, RAM 6c, hard disk 6d, input-output interface 6e, output interface 6f and communication interface 6g are connected via bus 6h to enable data communication thereamong.

CPU 6a can execute a computer program memorized in ROM 6b and hard disk 6d and a computer program read out by RAM 6c. Accordingly, the forward scattered light signal, side scattered light signal and fluorescence signal inputted from the measurement unit 1, specifically from the flow cytometer, are subjected to arithmetic processing with an installed program thereby identifying cells groups such as the first cell group and the second cell group and further counting hematopoietic progenitor cells.

A computer program for running CPU 6a, data and others used in executing the computer program, are memorized in ROM 6b. RAM 6c serves as a work area for CPU 6a in readout of a computer program memorized in ROM 6b and hard disk 6d and in execution of the computer program. A computer program for running CPU 6a, data and others used in executing the computer program, are memorized in the hard disk 6d. This computer program fulfills a function to analyze optical information and output analysis results.

A keyboard 8 is connected to the input-output interface 6e. The keyboard 8 serving as an input unit is arranged for operation or others on an outputted image. The output interface 6f is connected to an output unit 7 consisting of a liquid crystal display. The output unit 7 is arranged for outputting and displaying analysis results obtained in the control unit 6. The communication interface 6g is connected to the measurement unit 1 and fulfils a function to receive optical information.

Now, an illustrative embodiment of information processing in the control unit 6 is described.

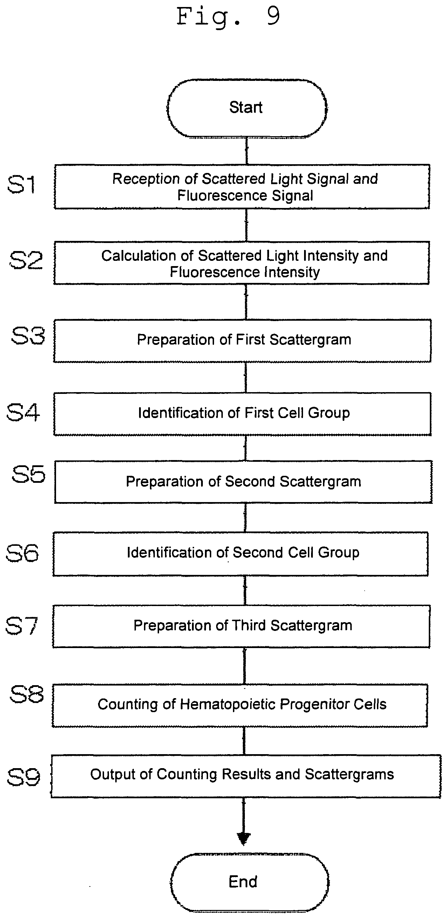

Fig. 9 is one example of a flowchart showing information processing corresponding to the first measurement method of the present invention. The control unit 6 receives, via a signal cable 3, the forward scattered light signal, side scattered light signal and fluorescence signal detected in the measurement unit 1 (step S1). The control unit 6 analyzes these signals and calculates the intensity of each signal (step S2). Then, the forward scattered light intensity and side scattered light intensity of concrete components (cells, platelets, for example) in the sample are used to prepare a first scattergram with the forward scattered light intensity and side scattered light intensity on the two axes (step S3). In the first scattergram, a hematopoietic progenitor cell candidate region where hematopoietic progenitor cells are estimated to appear is established, and a first cell group contained in this region is identified (step S4). The forward scattered light intensity and fluorescence intensity of concrete components in the sample are used to prepare a second scattergram with the forward scattered light intensity and fluorescence intensity on the two axes (step S5). In the second scattergram, a hematopoietic progenitor cell candidate region where hematopoietic progenitor cells are estimated to appear is established, and a second cell group contained in this region is identified (step S6). Then, the fluorescence intensity and side scattered light intensity of cells contained in the first cell group and also contained in the second cell group are used to prepare a third scattergram with the fluorescence intensity and side scattered light intensity on the two axes (step S7). The cells appearing in the third scattergram (cells contained in the first cell group and also contained in the second cell group) are counted as hematopoietic progenitor cells (step S8), and this counting result and the third scattergram are outputted in the output unit 7 (step S9).

In the flowchart in Fig. 9, steps S5 and S6 may be conducted not after steps S3 and S4 but before steps S3 and S4.

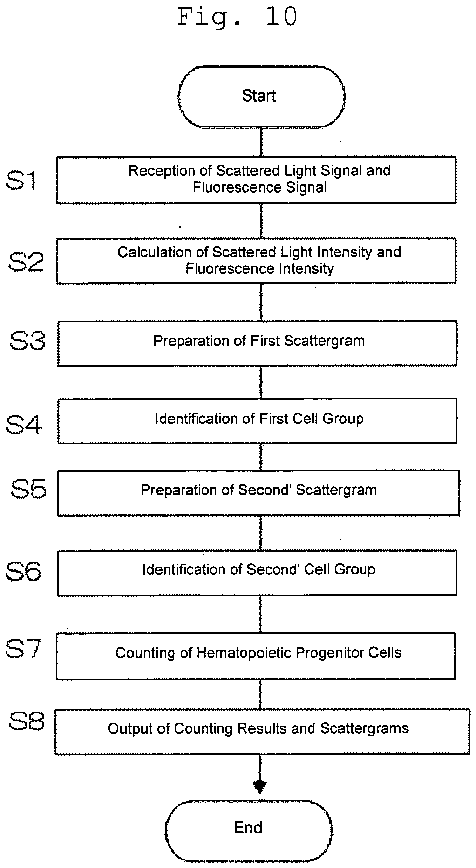

Fig. 10 is a flowchart showing information processing corresponding to the method for first identifying the first cell group in the second measurement method of the present invention. The control unit 6 receives, via the signal cable 3, the forward scattered light signal, side scattered light signal and fluorescence signal detected in the measurement unit 1 (step S1). The control unit 6 analyzes these signals and calculates the intensity of each signal (step S2). Then, the forward scattered light intensity and side scattered light intensity of concrete components (cells, platelets, for example) in the sample are used to prepare a first scattergram with the forward scattered light intensity and side scattered light intensity on the two axes (step S3). In the first scattergram, a hematopoietic progenitor cell candidate region where hematopoietic progenitor cells are estimated to appear is established, and a first cell group contained in this region is identified (step S4). The forward scattered light intensity and fluorescence intensity of the first cell group are used to prepare a scattergram (second' scattergram) with the forward scattered light intensity and fluorescence intensity on the two axes (step S5). In the second' scattergram, a hematopoietic progenitor cell candidate region where hematopoietic progenitor cells are estimated to appear is established, and a second' cell group contained in this candidate region is identified (step S6). The second' cell group is counted as hematopoietic progenitor cells (step S7), and this counting result and the second' scattergram are outputted in the output unit 7 (step S8).

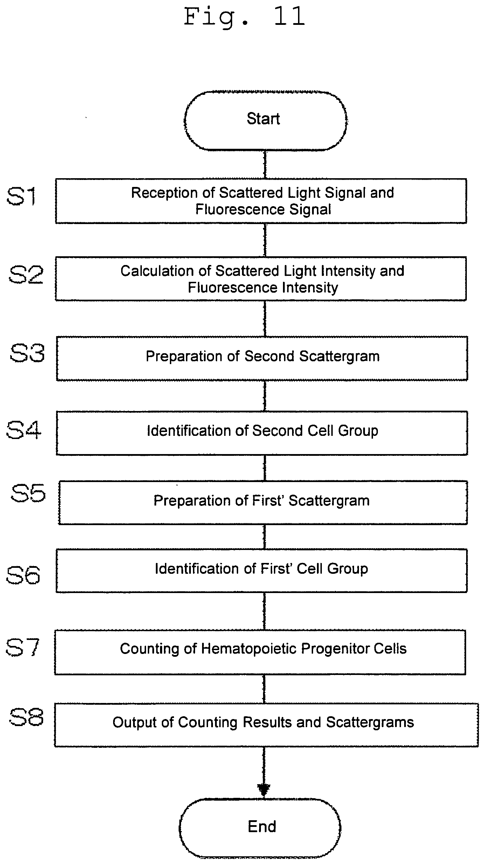

Fig. 11 is a flowchart showing information processing corresponding to the method for first identifying the second cell group in the second measurement method of the present invention. The control unit 6 receives, via the signal cable 3, the forward scattered light signal, side scattered light signal and fluorescence signal detected in the measurement unit 1 (step S1). The control unit 6 analyzes these signals and calculates the density of each signal (step S2). Then, the forward scattered light intensity and fluorescence intensity of concrete components (cells, platelets, for example) in the sample are used to prepare a second scattergram with the forward scattered light intensity and fluorescence intensity on the two axes (step S3). In the second scattergram, a hematopoietic progenitor cell candidate region where hematopoietic progenitor cells are estimated to appear is established, and a second cell group contained in this region is identified (step S4). Then, the forward scattered light intensity and side scattered light intensity of the second cell group are used to prepare a scattergram (first' scattergram) with the forward scattered light intensity and side scattered light intensity on the two axes (step S5). In the first' scattergram, a hematopoietic progenitor cell candidate region where hematopoietic progenitor cells are estimated to appear is established, and a first' cell group contained in this candidate region is identified (step S6). The first' cell group is counted as hematopoietic progenitor cells (step S7), and this counting result and the first' scattergram are outputted in the output unit 7 (step S8).

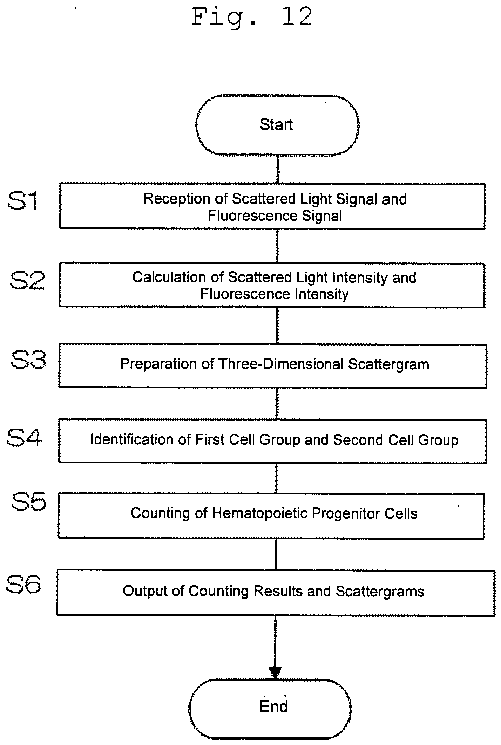

Fig. 12 is a flowchart showing information processing corresponding to the third measurement method of the present invention. The control unit 6 receives, via the signal cable 3, the forward scattered light signal, side scattered light signal and fluorescence signal detected in the measurement unit 1 (step S1). The control unit 6 analyzes these signals and calculates the intensity of each signal (step S2). Then, a three-dimensional scattergram with the forward scattered light intensity, side scattered light intensity and fluorescence intensity of concrete components (cells, platelets, for example) in the sample on the three axes is prepared (step S3). In this three-dimensional scattergram, a first cell group based on the forward scattered light intensity and side scattered light intensity, and a second cell group based on the forward scattered light intensity and fluorescence intensity, are identified (step S4). A hematopoietic progenitor cell candidate region where cells contained in the first cell group and also contained in the second group is established in this three-dimensional scattergram, and cells appearing in this region are counted as hematopoietic progenitor cells (step S5). The counting result of the hematopoietic progenitor cells, and the three-dimensional scattergram, are outputted in the output unit 7 (step S8).

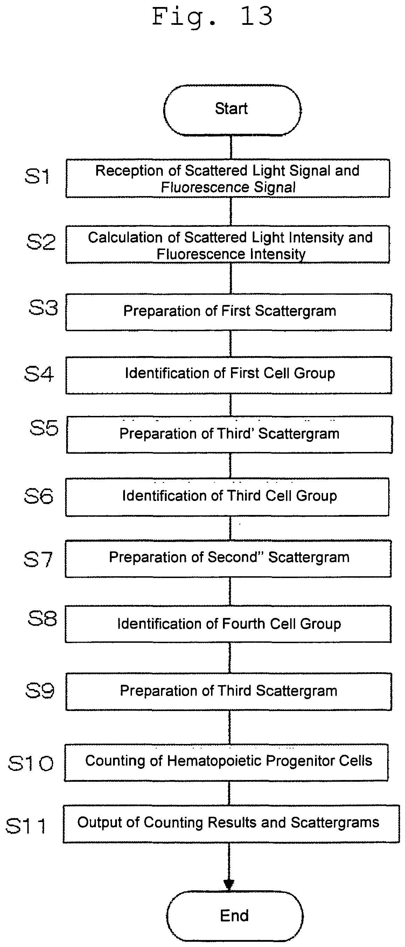

Fig. 13 is a flowchart showing information processing corresponding to the fourth measurement method of the present invention. The control unit 6 receives, via the signal cable 3, the forward scattered light signal, side scattered light signal and fluorescence signal detected in the measurement unit 1 (step S1). The control unit 6 analyzes these signals and calculates the intensity of each signal (step S2). Then, the forward scattered light intensity and side scattered light intensity of concrete components (cells, platelets, for example) in the sample are used to prepare a first scattergram with the forward scattered light intensity and side scattered light intensity on the two axes (step S3). In the first scattergram, a hematopoietic progenitor cell candidate region where hematopoietic progenitor cells are estimated to appear is established, and a first cell group contained in this region is identified (step S4). Then, the forward scattered light intensity and fluorescence intensity of concrete components in the sample are used to prepare a third' scattergram with the forward scattered light intensity and fluorescence intensity on the two axes (step S5). In the third' scattergram, a leukocyte appearing region where essentially all leukocytes are estimated to appear is established, and a third cell group contained in this region is identified (step S6). Further, the side scattered light intensity and fluorescence intensity of the third cell group are used to prepare a second" scattergram with the side scattered light intensity and fluorescence intensity on the two axes (step S7). In the second" scattergram, a hematopoietic progenitor cell candidate region where hematopoietic progenitor cells are estimated to appear is established, and a fourth cell group contained in this region is identified (step S8). Then, the fluorescence intensity and side scattered light intensity of cells contained in the first cell group and also contained in the fourth cell group are used to prepare a third scattergram with the fluorescence intensity and side scattered light intensity on the two axes (step S9). The cells appearing in the third scattergram (cells contained in the first cell group and also contained in the fourth cell group) are counted as hematopoietic progenitor cells (step S10), and this counting result and the third scattergram are outputted in the output unit 7 (step S11).

Steps S5 to S9 may be conducted not after steps S3 and S4 but before steps S3 and S4.

In the apparatus in the illustrative embodiments described above, the counting results of hematopoietic progenitor cells and scattergrams used in counting are outputted and displayed on the display of the output unit 7 by the control unit 6, but some of the counting results and/or the scattergrams may be outputted. When scattergrams are to be outputted, some of the scattergrams may be outputted, or all the prepared scattergrams may be outputted.

The "counting results of hematopoietic progenitor cells" in the embodiments described above include the number of hematopoietic progenitor cells in a sample, the number (concentration) of hematopoietic progenitor cells per unit volume, and the ratio of hematopoietic progenitor cells to all leukocytes, and at least one of these results can be outputted by the control unit 6.

### EXAMPLES

### Example 1

### Biological Samples

180 peripheral blood samples collected from 180 humans were used as biological samples (also referred to hereinafter as "the samples").

### Treating Reagent

A hemolytic agent A and a dyeing solution A, containing the following components, were used.

**Hemolytic agent A**

| | |
|---|---|
| Glycine | 15.7 g/lL |
| Polyoxyethylene (16) oleyl ether | 25000 ppm |
| N-lauroyl sarcosine sodium | 750 ppm |
| HEPES | 50 mM |
| Purified water | 1 l |
| Sodium hydroxide | Amount to adjust pH to 7.0 |

**Dyeing Solution A**

| | |
|---|---|
| Propidium iodide | 20 ppm |
| Ethylene glycol | 1 L |

980 µL of the hemolytic agent A, 20 µL of the dyeing solution A and 20 µL of the sample were mixed and then reacted at 33°C for 7 seconds to prepare a measurement sample. This measurement sample was introduced into a flow cytometer (light source: red semiconductor laser emitting a light at 633 nm) having the structure shown in Fig. 7, and measured for its forward scattered light intensity, side scattered light intensity and fluorescence intensity by irradiating concrete components in the measurement sample with a light.

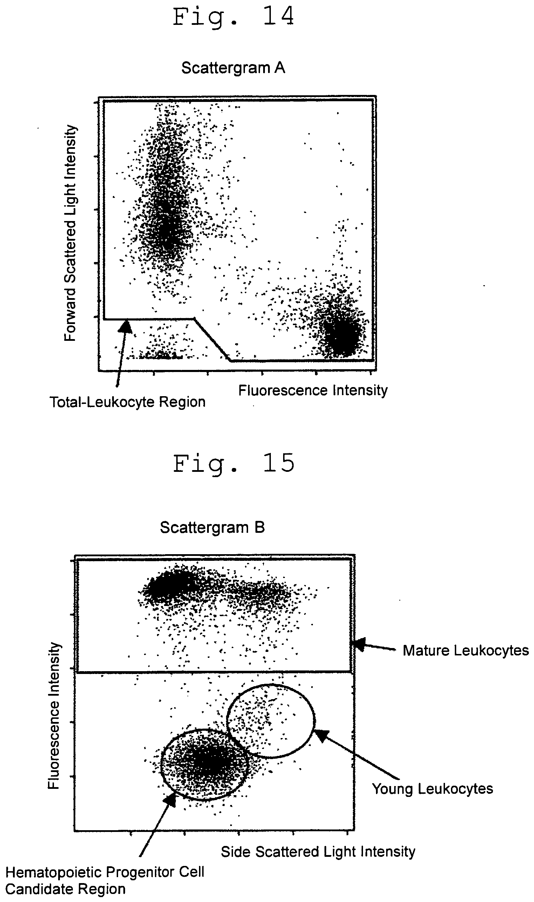

A scattergram A with the obtained forward scattered light intensity and fluorescence intensity on the two axes was prepared. A total-leukocyte region substantially free of a ghost was established in the scattergram A. Cells in this total-leukocyte region were developed in a graph with the side scattered light intensity and fluorescence intensity on the two axes, to prepare a scattergram B. The scattergrams A and B prepared in measuring certain measurement samples are shown in Figs. 14 and 15, respectively. In the scattergram B, a region of low side scattered light intensity and low fluorescence intensity was established as a hematopoietic progenitor cell candidate region (see Fig. 15). Cells appearing in this region were counted as hematopoietic progenitor cells, and the ratio of hematopoietic progenitor cells to the cells appearing in the total-leukocyte region (the number of substantially all leukocytes in the sample) was calculated.

### Comparative Example 1

As a control, the same biological sample as used in Example 1 was measured for its CD34-positive cells by using an anti-CD34 monoclonal antibody. CD34-positive cells measured in this comparative example and detected with an anti-CD34 monoclonal antibody correspond to hematopoietic progenitor cells.

20 µL of the sample was mixed with 5 µL of a labeled antibody solution in which an anti-CD34 monoclonal antibody labeled with fluorescein isothiocyanate (FITC) had been contained at a concentration of 0.0025 mg/mL, and the mixture was incubated at room temperature for 20 minutes. 2 mL of the hemolytic agent (containing 0.899 mg/mL ammonium chloride) was added thereto, and the mixture was reacted for 5 minutes to lyse erythrocytes in the sample. Then, the sample was centrifuged at 1000 rpm for 10 minutes, whereby leukocytes as a subject of measurement were precipitated, and the supernatant was discarded. 1 mL of PBS (pH 7.2) was added to the precipitated leukocytes, then the leukocytes were re-suspended and used as a measurement sample to count its total leukocytes and CD34-positive cells with a general-purpose flow cytometer FACSCalibur manufactured by Becton Dickinson, to calculate the ratio of hematopoietic progenitor cells to the total leukocytes.

### Results

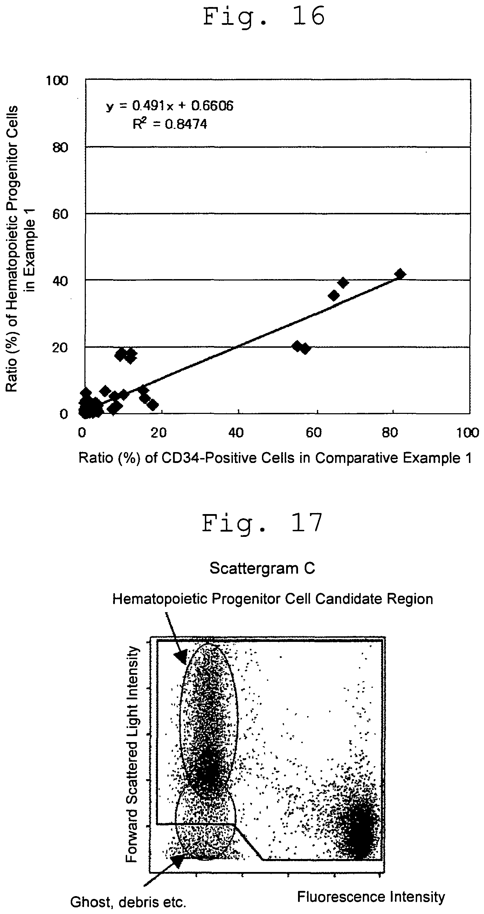

A graph showing the correlation between the ratio of hematopoietic progenitor cells calculated in Example 1 and the ratio of hematopoietic progenitor cells calculated in Comparative Example 1 is shown in Fig. 16.

As can be seen from Fig. 16, the measurement result obtained by using the conventional general-purpose flow cytometer (measurement result in Comparative Example 1) is well correlated with the measurement result in Example 1. From the foregoing, it was revealed that hematopoietic progenitor cells can be measured accurately according to the method in this example.

### Example 2

### Biological Samples

180 peripheral blood samples collected from 180 humans were used as biological samples (also referred to hereinafter as "the samples").

### Treating Reagent

A hemolytic agent B and a dyeing solution B, containing the following components, were used.

**Hemolytic agent B**

| | |
|---|---|
| Alanine | 19.8 g/lL |
| Polyoxyethylene (16) oleyl ether | 25000 ppm |
| N-lauroyl sarcosine sodium | 750 ppm |
| HEPES | 50 mM |
| Purified water | 1 l |
| Sodium hydroxide | Amount to adjust pH to 7.0 |

**Dyeing Solution B**

| | |
|---|---|
| Dye A | 50 ppm |
| Ethylene glycol | 1 L |

980 µL of the hemolytic agent B, 20 µL of the dyeing solution B and 20 µL of the sample were mixed and then reacted at 33°C for 7 seconds to prepare a measurement sample. This measurement sample was introduced into the flow cytometer used in Example 1 and measured for its forward scattered light intensity, side scattered light intensity and fluorescence intensity by irradiating concrete components in the measurement sample with a light.

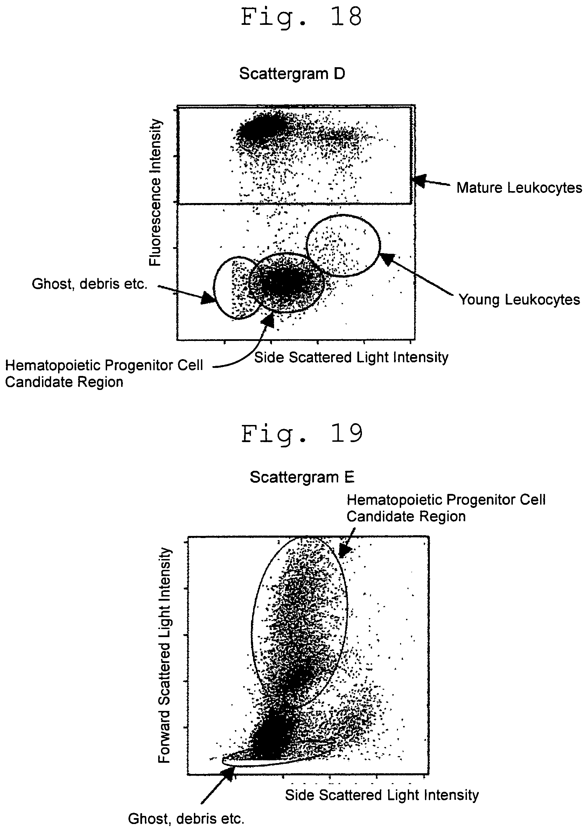

A scattergram C with the obtained forward scattered light intensity and fluorescence intensity on the two axes was prepared. A total-leukocyte region substantially free of a ghost was established in the scattergram C. Cells in this total-leukocyte region were developed in a graph with the side scattered light intensity and fluorescence intensity on the two axes, to prepare a scattergram D. The scattergrams C and D prepared in examining certain measurement samples are shown in Figs. 17 and 18, respectively. In the scattergram D, a region of low side scattered light intensity and low fluorescence intensity was established as a hematopoietic progenitor cell candidate region (see Fig. 18).

A scattergram E with the obtained forward scattered light intensity and side scattered light intensity on the two axes was prepared. The scattergram E prepared in examining the measurement sample is shown in Fig. 19.

In the scattergram E, a region of moderate side scattered light intensity and strong forward scattered light intensity was established as a hematopoietic progenitor cell candidate region (see Fig. 19).

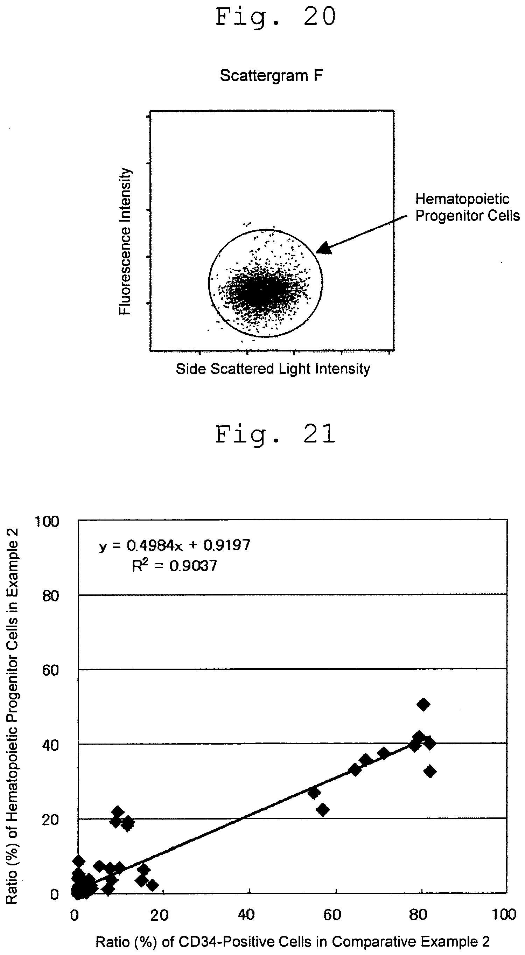

Cells appearing in the hematopoietic progenitor cell candidate region in the scattergram D and also appearing in the hematopoietic progenitor cell candidate region in the scattergram E were developed in a graph on the basis of the fluorescence intensity and side scattered light intensity, to prepare a scattergram F. The scattergram F prepared in examining the measurement sample is shown in Fig. 20. The cells appearing in the scattergram F were counted as hematopoietic progenitor cells, to calculate the ratio of hematopoietic progenitor cells to the cells appearing in the total-leukocyte region (the number of substantially all leukocytes in the sample).

### Comparative Example 2

Hematopoietic progenitor cells were measured and the ratio of hematopoietic progenitor cells to total leukocytes was calculated in the same manner as in Comparative Example 1 except that the samples used in Example 2 were used.

### Results

A graph showing the correlation between the ratio of hematopoietic progenitor cells calculated in Example 2 and the ratio of hematopoietic progenitor cells calculated in Comparative Example 2 is shown in Fig. 21.

As can be seen from Fig. 21, the measurement result obtained by using the conventional general-purpose flow cytometer (measurement result in Comparative Example 2) is well correlated with the measurement result in Example 2. From the foregoing, it was revealed that hematopoietic progenitor cells can be measured accurately according to the method in this example.

### Example 3

11 blood samples were collected from a subject A at predetermined time intervals, and these 11 samples were used as those from the subject A. Separately, 11 blood samples were collected from a subject B at predetermined time intervals, and these 11 samples were used as those from the subject B.

These samples were measured for their hematopoietic progenitor cells in the same manner as in Example 1 except that a hemolytic agent C and dyeing solution C containing the following compositions were used. The ratio of hematopoietic progenitor cells to total leukocytes was calculated.

**Hemolytic Agent C**

| | |
|---|---|
| Xylitol | 35.5 g/lL |
| Polyoxyethylene (16) oleyl ether | 20000 ppm |
| N-lauroyl sarcosine sodium | 750 ppm |
| HEPES | 30 mM |
| Purified water | 1 l |
| Sodium hydroxide | Amount to adjust pH to 7.0 |

**Dyeing Solution C**

| | |
|---|---|
| Dye A | 20 ppm |
| Ethylene glycol | 1 L |

### Comparative Example 3

Hematopoietic progenitor cells were measured and the ratio of hematopoietic progenitor cells to total leukocytes was measured in the same manner as in Comparative Example 1 except that the samples used in Example 3 were used.

### Results

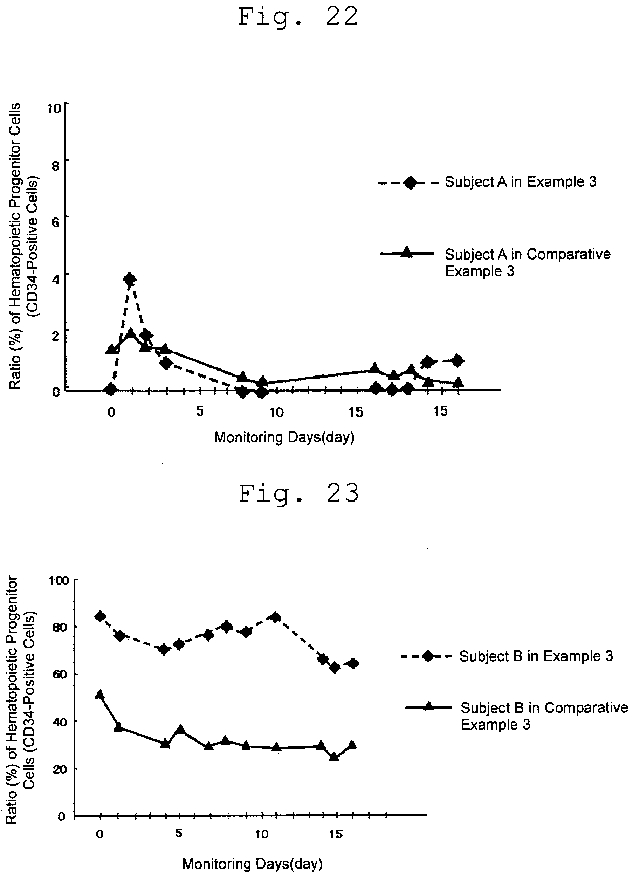

In Example 3 and Comparative Example 3, the measurement results for the subj ect A are shown in Fig. 21, and the measurement results for the subject B are shown in Fig. 22. Figs. 22 and 23 are graphs showing the results of monitoring a fluctuation in hematopoietic progenitor cells in the blood samples collected 11 times for a predetermined period of time from Day 0 as the start date of blood collection. As can be seen from Figs. 22 and 23, the measurement results in Example 3 are almost connected with the measurement results by the conventionally used method (measurement results in Comparative Example 3). From the foregoing, it was revealed that hematopoietic progenitor cells can be accurately measured according to the method in Example 3.

## Claims

1. A method for measuring a biological sample, comprising steps of:
preparing a measurement sample by damaging cell membranes of erythrocytes and mature leukocytes in a biological sample, substantially not damaging cell membranes of hematopoietic progenitor cells in the biological sample, shrinking the damaged erythrocytes, and staining nucleic acid in the damaged mature leukocytes with a fluorescent dye by mixing a surfactant for damaging cell membranes of erythrocytes and mature leukocytes, a solubilizing agent for shrinking the damaged erythrocytes, a fluorescent dye for staining a nucleic acid, and a biological sample for 3 seconds to 5 minutes, wherein the surfactant is a polyoxyethylene non-ionic surfactant represented by the formula: R¹-R²-(CH₂CH₂O)ₙ-H, wherein R¹ is a C₉ to C₂₅ alkyl, alkenyl or alkynyl group, R² is -O-, -COO- or 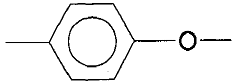 and n is an integer of 10 to 40;
irradiating cells in the measurement sample with light;
obtaining forward scattered light intensity, side scattered light intensity and fluorescence intensity from the irradiated cells;
specifying hematopoietic progenitor cells based on the obtained forward and side scattered light intensities and the obtained fluorescence intensity, comprising a first preparation of a first two-dimensional scattergram and a second preparation of a second two-dimensional scattergram; and
counting the specified hematopoietic progenitor cells;
**characterized in that**:
the first two dimensional scattergram has the forward and side scattered light intensities on the two axes and displays hematopoietic progenitor cells and platelet aggregate discriminated by the forward and side scattered light intensities; and
the second two dimensional scattergram has the forward or side scattered light intensity and the fluorescent intensity on the two axes and displays hematopoietic progenitor cells, granulocytes, lymphocytes, and monocytes discriminated by the forward or side scattered light intensity and the fluorescence intensity.

2. A method according to claim 1, wherein the specifying step is performed by:
determining a first cell group containing hematopoietic progenitor cells based on the forward and side scattered light intensities;
determining a second cell group containing hematopoietic progenitor cells based on the forward scattered light intensity and the fluorescence intensity; and
specifying, as hematopoietic progenitor cells, cells which belong to both of the first and second cell groups.

3. A method according to claim 1, which further comprises steps of:
displaying the first and second scattergrams.

4. A method according to claim 1, wherein the specifying step is performed by:
determining a first cell group containing hematopoietic progenitor cells based on the forward and side scattered light intensities; and
specifying hematopoietic progenitor cells from among the first cell group, on the basis of the forward scattered light intensity and fluorescence intensity of the first cell group.

5. A method according to claim 1, wherein the specifying step is performed by:
determining a first cell group containing hematopoietic progenitor cells based on the forward scattered light intensity and fluorescence intensity;
specifying hematopoietic progenitor cells from among the first cell group, on the basis of the forward and side scattered light intensities of the first cell group.

6. A method according to claim 2, wherein the second two-dimensional scattergram has the forward scattered light intensity and the fluorescence intensity on the two axes and displays a second cell group appearing region including hematopoietic progenitor cells on the basis of the forward scattered light intensity and the fluorescence intensity.

7. A method according to claim 1, wherein the specifying step is performed by
determining a first cell group containing hematopoietic progenitor cells, based on the forward and side scattered light intensities;
determining, as a second cell group, a cell group other than a ghost group based on the forward scattered light intensity and the fluorescence intensity;
determining a third cell group substantially free of at least mature leukocytes, from among the second cell group, based on the side scattered light intensity and the fluorescence intensity; and
specifying, as hematopoietic progenitor cells, cells which belong to both of the first and third cell groups.

8. A method according to claim 1, wherein the sample preparing step is performed by mixing the biological sample and a reagent containing a surfactant for damaging cell membranes of the erythrocytes and mature leukocytes, and a solubilizing agent for shrinking the damaged erythrocytes thereby damaging cell membranes of the erythrocytes and mature leukocytes contained in the biological sample and shrinking the damaged erythrocytes.

9. A method according to claim 1 wherein the sample preparing step is performed by mixing the biological sample and a reagent containing a surfactant for damaging cell membranes of the erythrocytes and mature leukocytes, a solubilizing agent for shrinking the damaged erythrocytes, and a sugar thereby damaging cell membranes of the erythrocytes and mature leukocytes contained in the biological sample and shrinking the damaged erythrocytes.

10. A method according to claim 9, wherein the sugar is at least one member selected from the group consisting of xylitol, arabinose, glucose, mannitol, sorbitol and ribitol.

## Patentansprüche

1. Verfahren zum Messen einer biologischen Probe, umfassend die Schritte:
Bereitstellen einer Messungsprobe durch Beschädigen von Zellmembranen von Erythrozyten und reifen Leukozyten in einer biologischen Probe, im Wesentlichen kein Beschädigen von Zellmembranen von hämatopoetischen Vorläuferzellen in der biologischen Probe, Schrumpfen der beschädigten Erythrozyten und Anfärben von Nukleinsäure in den beschädigten reifen Leukozyten mit einem Fluoreszenzfarbstoff, indem eine oberflächenaktive Substanz zum Beschädigen von Zellmembranen von Erythrozyten und reifen Leukozyten, ein Lösungsmittel zum Schrumpfen der beschädigten Erythrozyten, ein Fluoreszenzfarbstoff zum Anfärben einer Nukleinsäure und eine biologische Probe für 3 Sekunden bis 5 Minuten gemischt werden, wobei die oberflächenaktive Substanz eine nicht-ionische oberflächenaktive Polyoxyethylen-Substanz ist, dargestellt durch die Formel: R¹-R²-(CH₂CH₂O)ₙ-H, wobei R¹ eine C₉ bis C₂₅ Aklyl-, Alkenyl-oder Alkynyl-Gruppe ist, R² -O-, -COO- oder 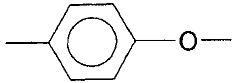 ist, und n eine Ganzzahl von 10 bis 40 ist;
Bestrahlen von Zellen in der Messungsprobe mit Licht;
Erhalten der Intensität von Vorwärtsstreulicht, der Intensität von Seitwärtsstreulicht und Fluoreszenzintensität aus den bestrahlten Zellen; Bestimmen von hämatopoetischen Vorläuferzellen basierend auf den erhaltenen Intensitäten des Vorwärts- und Seitwärtsstreulichts und der erhaltenen Fluoreszenzintensität, umfassend ein erstes Bereitstellen eines ersten zweidimensionalen Streudiagramms und ein zweites Bereitstellen eines zweiten zweidimensionalen Streudiagramms; und
Zählen der bestimmten hämatopoetischen Vorläuferzellen;
charakterisiert dadurch, dass:
das erste zweidimensionale Streudiagramm die Intensitäten des Vorwärts- und Seitwärtsstreulichts auf den zwei Achsen hat und hämatopoetische Vorläuferzellen und Blutplättchenaggregat anzeigt, die durch die Intensitäten des Vorwärts- und Seitwärtsstreulichts unterschieden werden; und
das zweite zweidimensionale Streudiagramm die Intensität des Vorwärts- oder Seitwärtsstreulichts und die Fluoreszenzintensität auf den zwei Achsen hat und hämatopoetische Vorläuferzellen, Granulozyten, Lymphozyten und Monozyten anzeigt, die durch die Intensität des Vorwärts- oder Seitwärtsstreulichts und der Fluoreszenzintensität unterschieden werden.

2. Verfahren gemäß Anspruch 1, wobei der Schritt des Bestimmens durchgeführt wird durch:
Ermitteln einer ersten Zellgruppe, die hämatopoetische Vorläuferzellen enthält, basierend auf den Intensitäten des Vorwärts- und Seitwärtsstreulichts;
Ermitteln einer zweiten Zellgruppe, die hämatopoetische Vorläuferzellen enthält, basierend auf der Intensität des Vorwärtsstreulichts und der Fluoreszenzintensität; und
Bestimmen von Zellen, die sowohl zur ersten als auch zur zweiten Zellgruppe gehören, als hämatopoetische Vorläuferzellen.

3. Verfahren gemäß Anspruch 1, das weiterhin die Schritte umfasst:
Anzeigen des ersten und zweiten Streudiagramms.

4. Verfahren gemäß Anspruch 1, wobei der Schritt des Bestimmens durchgeführt wird durch:
Ermitteln einer ersten Zellgruppe, die hämatopoetische Vorläuferzellen enthält, basierend auf den Intensitäten des Vorwärts- und Seitwärtsstreulichts;
Bestimmen von hämatopoetischen Vorläuferzellen aus der ersten Zellgruppe auf Basis der Intensität des Vorwärtsstreulichts und der Fluoreszenzintensität der ersten Zellgruppe.

5. Verfahren gemäß Anspruch 1, wobei der Schritt des Bestimmens durchgeführt wird durch:
Ermitteln einer ersten Zellgruppe, die hämatopoetische Vorläuferzellen enthält, basierend auf der Intensität des Vorwärtsstreulichts und der Fluoreszenzintensität;
Bestimmen von hämatopoetischen Vorläuferzellen aus der ersten Zellgruppe auf Basis der Intensitäten des Vorwärts- und Seitwärtsstreulichts der ersten Zellgruppe.

6. Verfahren gemäß Anspruch 2, wobei das zweite zweidimensionale Streudiagramm die Intensität des Vorwärtsstreulichts und die Fluoreszenzintensität auf den zwei Achsen hat und eine Erschein-Region der zweiten Zellgruppe, die hämatopoetische Vorläuferzellen auf Basis der Intensität des Vorwärtsstreulichts und der Fluoreszenzintensität einschließt, anzeigt.

7. Verfahren gemäß Anspruch 1, wobei der Schritt des Bestimmens durchgeführt wird durch:
Ermitteln einer ersten Zellgruppe, die hämatopoetische Vorläuferzellen enthält, basierend auf den Intensitäten des Vorwärts- und Seitwärtsstreulichts;
Ermitteln einer Zellgruppe, die keine Ghost-Gruppe ist, als eine zweite Zellgruppe, basierend auf der Intensität des Vorwärtsstreulichts und der Fluoreszenzintensität;
Ermitteln einer im Wesentlichen wenigstens von reifen Leukozyten freien dritten Zellgruppe aus der zweiten Zellgruppe, basierend auf der Intensität des Seitwärtsstreulichts und der Fluoreszenzintensität; und
Bestimmen von Zellen, die sowohl zur ersten als auch zur dritten Zellgruppe gehören, als hämatopoetische Vorläuferzellen.

8. Verfahren gemäß Anspruch 1, wobei der Schritt des Bereitstellens der Probe durchgeführt wird durch Mischen der biologischen Probe mit einem Reagenz, das eine oberflächenaktive Substanz zum Beschädigen von Zellmembranen der Erythrozyten und reifen Leukozyten und ein Lösungsmittel zum Schrumpfen der beschädigten Erythrozyten enthält, wodurch Zellmembranen der Erythrozyten und reifen Leukozyten in der biologischen Probe beschädigt und die beschädigten Erythrozyten geschrumpft werden.

9. Verfahren gemäß Anspruch 1, wobei der Schritt des Bereitstellens der Probe durchgeführt wird durch Mischen der biologischen Probe mit einem Reagenz, das eine oberflächenaktive Substanz zum Beschädigen von Zellmembranen der Erythrozyten und reifen Leukozyten, ein Lösungsmittel zum Schrumpfen der beschädigten Erythrozyten und einen Zucker enthält, wodurch Zellmembranen der in der biologischen Probe enthaltenen Erythrozyten und reifen Leukozyten beschädigt und die beschädigten Erythrozyten geschrumpft werden.

10. Verfahren gemäß Anspruch 9, wobei der Zucker wenigstens ein Mitglied ist, ausgewählt aus der Gruppe, bestehend aus Xylitol, Arabinose, Glukose, Mannitol, Sorbitol und Ribitol.

## Revendications

1. Procédé pour mesurer un échantillon biologique, comprenant les étapes consistant à :
préparer un échantillon de mesure en endommageant les membranes cellulaires d'érythrocytes et de leucocytes matures dans un échantillon biologique, essentiellement sans endommager les membranes cellulaires de cellules progénitrices hématopoïétiques dans l'échantillon biologique,
faire rétrécir les érythrocytes endommagés, et colorer un acide nucléique dans les leucocytes matures endommagés avec un colorant fluorescent en mélangeant un tensioactif pour endommager les membranes cellulaires d'érythrocytes et de leucocytes matures, un agent solubilisant pour faire rétrécir les érythrocytes endommagés, un colorant fluorescent pour colorer un acide nucléique, et un échantillon biologique pendant 3 secondes à 5 minutes, dans lequel le tensioactif est un tensioactif non-ionique de polyoxyéthylène représenté par la formule : R¹-R²-(CH₂CH₂O)ₙ-H, dans lequel R¹ est un groupe alkyle, alcényle ou alcynyle en C9 à C25, R² est un -O-, un -COO- ou 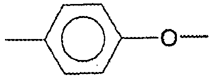 et n est un nombre entier de 10 à 40 ;
irradier des cellules dans l'échantillon de mesure avec de la lumière,
obtenir une intensité de lumière dispersée vers l'avant, une intensité de lumière dispersée latéralement et une intensité de fluorescence des cellules irradiées ;
spécifier des cellules progénitrices hématopoïétiques sur la base des intensités de lumière dispersée vers l'avant et latéralement obtenues et de l'intensité de fluorescence obtenue, comprenant une première préparation d'un premier scattergramme bidimensionnel et une deuxième préparation d'un deuxième scattergramme bidimensionnel ;
et
compter les cellules progénitrices hématopoïétiques spécifiées ;
**caractérisé en ce que** :
le premier scattergramme bidimensionnel a les intensités de lumière dispersée vers l'avant et latéralement sur les deux axes et affiche des cellules progénitrices hématopoïétiques et un agrégat de plaquettes distingués par les intensités de lumière dispersée vers l'avant et latéralement; et
le deuxième scattergramme bidimensionnel a l'intensité de lumière dispersée vers l'avant ou latéralement et l'intensité de fluorescence sur les deux axes et affiche des cellules progénitrices hématopoïétiques, des granulocytes, des lymphocytes, et des monocytes distingués par l'intensité de lumière dispersée vers l'avant ou latéralement et l'intensité de fluorescence.

2. Procédé selon la revendication 1, dans lequel l'étape d'identification est réalisée en :
déterminant un premier groupe de cellules contenant des cellules progénitrices hématopoïétiques sur la base des intensités de lumière dispersée vers l'avant et latéralement;
déterminant un deuxième groupe de cellules contenant des cellules progénitrices hématopoïétiques sur la base de l'intensité de lumière dispersée vers l'avant et de l'intensité de fluorescence ; et
spécifiant, en tant que cellules progénitrices hématopoïétiques, des cellules qui appartiennent aux deux des premier et deuxième groupes de cellules.

3. Procédé selon la revendication 1, qui comprend en outre les étapes consistant à :
afficher les premier et deuxième scattergrammes.

4. Procédé selon la revendication 1, dans lequel l'étape d'identification est réalisée en :
déterminant un premier groupe de cellules contenant des cellules progénitrices hématopoïétiques sur la base des intensités de lumière dispersée vers l'avant et latéralement; et
spécifiant des cellules progénitrices hématopoïétiques parmi le premier groupe de cellules, sur la base de l'intensité de lumière dispersée vers l'avant et de l'intensité de fluorescence du premier groupe de cellules.

5. Procédé selon la revendication 1, dans lequel l'étape d'identification est réalisée en :
déterminant un premier groupe de cellules contenant des cellules progénitrices hématopoïétiques sur la base de l'intensité de lumière dispersée vers l'avant et de l'intensité de fluorescence ;
spécifiant des cellules progénitrices hématopoïétiques parmi le premier groupe de cellules, sur la base des intensités de lumière dispersée vers l'avant et latéralement du premier groupe de cellules.

6. Procédé selon la revendication 2, dans lequel le deuxième scatergramme bidimensionnel a l'intensité de lumière dispersée vers l'avant et l'intensité de fluorescence sur les deux axes et affiche une région d'apparition d'un deuxième groupe de cellules incluant des cellules progénitrices hématopoïétiques sur la base de l'intensité de lumière dispersée vers l'avant et de l'intensité de fluorescence.

7. Procédé selon la revendication 1, dans lequel l'étape de spécification est réalisée en :
déterminant un premier groupe de cellules contenant des cellules progénitrices hématopoïétiques, sur la base des intensités de lumière dispersée vers l'avant et latéralement;
déterminant, en tant qu'un deuxième groupe de cellules, un groupe de cellules autre qu'un groupe fantôme sur la base de l'intensité de lumière dispersée vers l'avant et de l'intensité de fluorescence ;
déterminant un troisième groupe de cellules essentiellement libre au moins de leucocytes matures, parmi le deuxième groupe de cellules, sur la base de l'intensité de lumière dispersée latéralement et de l'intensité de fluorescence ; et
spécifiant, en tant que cellules progénitrices hématopoïétiques, des cellule qui appartiennent aux deux des premier et troisième groupes de cellules.

8. Procédé selon la revendication 1, dans lequel l'étape de préparation d'échantillon est réalisée en mélangeant l'échantillon biologique et un réactif contenant un tensioactif pour endommager les membranes cellulaires des érythrocytes et des leucocytes matures, et un agent solubilisant pour faire rétrécir les érythrocytes endommagés en endommageant ainsi les membranes cellulaires des érythrocytes et des leucocytes matures contenus dans l'échantillon biologique et en faisant rétrécir les érythrocytes endommagés.

9. Procédé selon la revendication 1, dans lequel l'étape de préparation d'échantillon est réalisée en mélangeant l'échantillon biologique et un réactif contenant un tensioactif pour endommager les membranes cellulaires des érythrocytes et des leucocytes matures, un agent solubilisant pour faire rétrécir les érythrocytes endommagés, et un sucre, en endommageant ainsi les membranes cellulaires des érythrocytes et des leucocytes matures contenus dans l'échantillon biologique et faisant rétrécir les érythrocytes endommagés.

10. Procédé selon la revendication 9, dans lequel le sucre est au moins un membre sélectionné dans le groupe constitué de xylitol, d'arabinose, de glucose, de mannitol, de sorbitol et de ribitol.
